(19) Europäisches Patentamt
European Patent Office
Office européen des brevets

(11) EP 0 754 693 A2

(12) DEMANDE DE BREVET EUROPEEN

(43) Date de publication:
**22.01.1997 Bulletin 1997/04**

(21) Numéro de dépôt: **96401594.5**

(22) Date de dépôt: **18.07.1996**

(51) Int Cl.[6]: **C07F 9/38**, A61K 31/66, C07F 9/572, C07F 9/40, C07F 9/655, C07F 9/6553

(84) Etats contractants désignés:
**AT BE CH DE DK ES FI FR GB GR IE IT LI LU NL PT SE**

(30) Priorité: **21.07.1995 FR 9508821**

(71) Demandeur: **ADIR ET COMPAGNIE**
**F-92415 Courbevoie Cédex (FR)**

(72) Inventeurs:
- **Cordi, Alex**
  **92150 Suresnes (FR)**
- **Desos, Patrice**
  **92400 Courbevoie (FR)**
- **Morris, Angela D.**
  **78180 Montigny Le Bretonneux (FR)**
- **Atassi, Ghanem**
  **92210 Saint Cloud (FR)**

# (54) Nouveaux dérivés d'acide aminophénylphosphonique, leur procédé de préparation et les compositions pharmaceutiques qui les contiennent

(57) Composé de formule (I) :

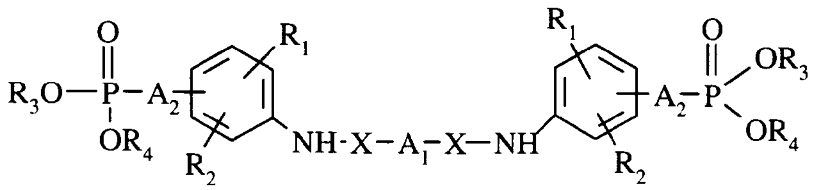

(I)

dans laquelle :

- $R_1$, $R_2$ identiques ou différents, représentent un atome d'hydrogène, d'halogène, un groupement alkyle, alkoxy, nitro ou trihalogénométhyle,
- X représente CO, $SO_2$ ou $CH_2$,
- $A_1$ représente l'un quelconque des groupements tels que définis dans la description,
- $A_2$ représente $-(CH_2)_n$ ou -CH=CH-,
- $R_3$, $R_4$, identiques ou différents, représentent un atome d'hydrogène ou un groupement alkyle,

ses isomères ainsi que ses sels d'addition à une base pharmaceutiquement acceptable.
Médicaments.

EP 0 754 693 A2

## Description

La présente invention concerne de nouveaux dérivés d'acide aminophénylphosphonique, leur procédé de préparation et les compositions pharmaceutiques qui les contiennent, utiles en tant qu'inhibiteurs d'angiogenèse et dans les pathologies où l'angiogénèse intervient.

L'angiogenèse (ou néovascularisation) est définie comme le développement et la croissance de nouveaux vaisseaux sanguins capillaires. Le processus d'angiogenèse est essentiel dans de nombreuses situations physiologiques dont le développement de l'embryon, la cicatrisation normale de blessures et le développement de l'endometrium après menstruation. En dehors de ces situations, l'angiogenèse chez l'adulte normal est très rare et la mitose des cellules endothéliales qui génère les parois des vaisseaux sanguins est très lente, avec des temps de renouvellement cellulaire mesurés en années.

Une angiogenèse anormale (c'est-à-dire la stimulation de la croissance de nouveaux vaisseaux sanguins due à un syndrome pathologique) est une caractéristique établie pour de nombreuses maladies, notamment la rétinopathie diabétique, l'arthrite rhumatoïde, les hémangiomes et la croissance de tumeurs solides ainsi que dans le développement des métastases.

Dans le domaine de l'oncologie, il a été montré que la croissance de tumeurs solides est tout à fait dépendante du développement constant de nouveaux vaisseaux sanguins et qu'il est corrélé, pour les métastases de certains cancers, avec la taille croissante de la tumeur primaire (J. Folkman, *New Engl. Med.,* 285 (1974), 1182-1185).

Un traitement pharmaceutique à l'aide d'un inhibiteur d'angiogenèse peut donc stopper la croissance de tumeurs primaires, empêcher ou réduire la formation de métastases, empêcher l'apparition de tumeurs secondaires. De tels inhibiteurs d'angiogenèse sont également utiles dans le traitement de maladies non-néoplasiques mentionnées précédemment dans lesquelles apparaît une activité angiogénique.

Les besoins de la thérapeutique exigent le développement constant de nouveaux composés inhibiteurs d'angiogenèse dans le but d'obtenir des principes actifs à la fois plus actifs, plus spécifiques et moins toxiques.

Parmi les inhibiteurs d'angiogénèse, la suramine est un exemple bien connu. D'autre part, des dérivés de la suramine ont été étudiés dans la littérature, c'est le cas notamment des composés décrits par K.D. Jentsch et coll (*J. Gen. Virol.,* 68, 2183-2192, 1987) ou dans le brevet WO 90/15816.

Les composés de la présente invention, outre le fait qu'ils soient nouveaux, présentent l'avantage d'être particulièrement puissants sans présenter de cytotoxicité particulière, à la différence de la suramine et de ses dérivés décrits dans l'Art Antérieur. De plus, les composés de l'invention présentent une activité non négligeable sur le cycle reproductif du virus du SIDA.

L'invention concerne plus spécifiquement les composés de formule (I) :

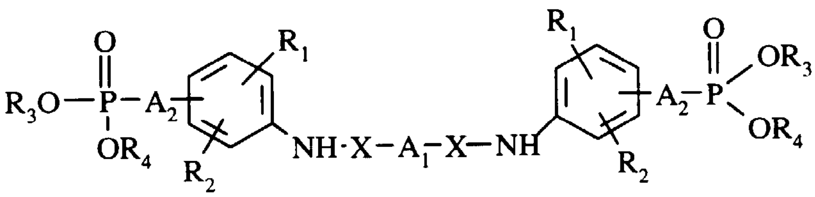

(I)

dans laquelle :

- $R_1$, $R_2$ identiques ou différents, représentent un atome d'hydrogène, d'halogène, un groupement alkyle ($C_1$-$C_6$) linéaire ou ramifié, un groupement alkoxy ($C_1$-$C_6$) linéaire ou ramifié, un groupement nitro ou un groupement trihalogénométhyle,
- X représente CO, $SO_2$ ou $CH_2$,
- $A_1$ représente une chaîne alkylène ($C_1$-$C_{20}$) linéaire ou ramifiée comportant de 0 à 6 doubles liaisons et dans laquelle un ou plusieurs groupements $-CH_2-$ de la chaîne sont éventuellement remplacés par l'un quelconque des groupements suivants :
  - phénylène substitué ou non,
  - naphtylène substitué ou non,
  - anthracénylène substitué ou non,
  - cycloalkylène ($C_3$-$C_7$),

- 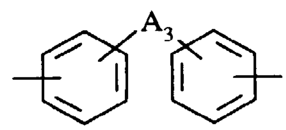

  dans lequel $A_3$ représente $-(CH_2)_m$
  (dans lequel m représente 0, 1 ou 2), -(CH = CH)- ou $SO_2$,

- 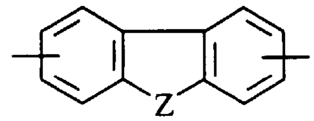

  dans lequel Z représente O, S ou $NR_5$
  (dans lequel $R_5$ représente un atome d'hydrogène ou un groupement alkyle ($C_1$-$C_6$) linéaire ou ramifié),

- 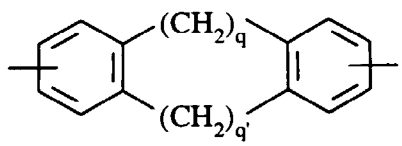

  dans lequel q, q', identiques ou différents, représentent 0, 1 ou2,

- 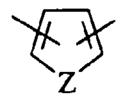

  dans lequel Z est tel que défini précédemment,

$A_2$ représente :

- $-(CH_2)_n-$ dans lequel n est égal à 0, 1, 2 ou 3,
- ou, -CH=CH-,

$R_3$, $R_4$, identiques ou différents, représentent un atome d'hydrogène ou un groupement alkyle ($C_1$-$C_6$) linéaire ou ramifié,

leurs isomères ainsi que leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

Par groupement phénylène substitué ou non, napthylène substitué ou non ou anthracénylène substitué ou non, on entend éventuellement substitué par un ou plusieurs atomes d'halogène ou groupements alkyle ($C_1$-$C_6$) linéaire ou ramifié, trihalogénoalkyle ($C_1$-$C_6$) linéaire ou ramifié, alkoxy ($C_1$-$C_6$) linéaire ou ramifié, hydroxy, nitro, cyano ou amino (substitué éventuellement par un ou plusieurs groupements alkyle ($C_1$-$C_6$) liénaire ou ramifié).

L'invention s'étend également au procédé de préparation du composé de formule (I) caractérisé en ce que l'on utilise comme produit de départ une amine de formule (II) :

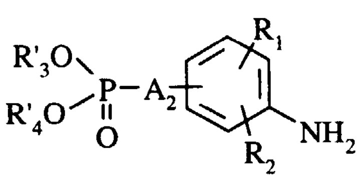

(II)

dans lequel $R_1$, $R_2$ et $A_2$ ont la même signification que dans la formule (I) et $R'_3$, $R'_4$, identiques ou différents, représentent un groupement alkyle ($C_1$-$C_4$) linéaire ou ramifié, que l'on fait réagir en milieu basique sur 1/2 équivalent d'halogénure de formule (III) :

$$Hal - X - A_1 - X - Hal \qquad (III)$$

dans laquelle Hal représente un atome d'halogène, X et $A_1$ ont la même signification que dans la formule (I), pour conduire au diester de formule (I/a), cas particulier des composés de formule (I) :

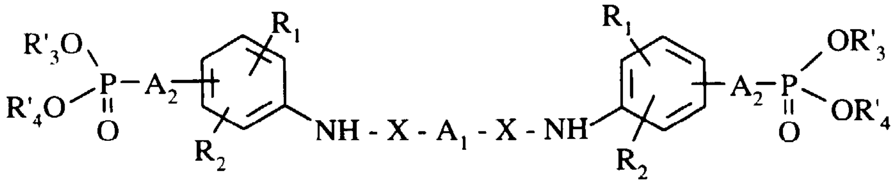

(I/a)

dans laquelle $R_1$, $R_2$, $R'_3$, $R'_4$, X, $A_1$ et $A_2$ sont tels que définis précédemment,
que l'on transforme en monoester correspondant en milieu acide, cas particulier des composés de formule (I),
ou en acide phosphonique correspondant en présence de bromure de triméthylsilyle, cas particulier des composés de formule (I),
diester, monoester ou acide phosphonique,

- qui peut être, le cas échéant, purifié selon une technique classique de purification,
- dont on sépare, le cas échéant, les isomères selon une technique classique de séparation,
- que l'on transforme, si on le souhaite, en ses sels d'addition à un acide ou à une base pharmaceutique acceptable.

Les composés de formule (II) sont obtenus à partir de l'amine de formule (V) :

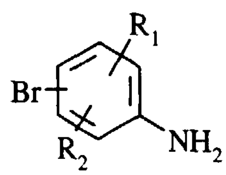

(V)

dans laquelle $R_1$ et $R_2$ ont la même signification que dans la formule (I), que l'on protège à l'aide d'un groupement protecteur tel que le bromure de benzyle en milieu basique, pour conduire au composé de formule (VI) :

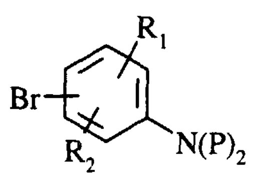

(VI)

dans laquelle $R_1$ et $R_2$ ont la même signification que dans la formule (I) et P représente un groupement protecteur, que l'on fait réagir avec un dialkylphosphite en présence d'un catalyseur, pour conduire au composé de formule (VII) :

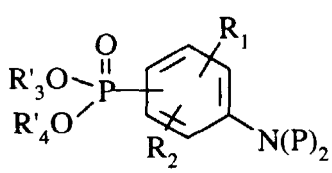

(VII)

dans laquelle $R_1$, $R_2$ et P ont la même signification que dans la formule (VI) et $R'_3$, $R'_4$, identiques ou différents, représentent un groupement alkyle ($C_1$ -$C_4$) linéaire ou ramifié,
dont on déprotège la fonction amine, pour conduire au composé de formule (II) correspondant.

Les substituants $R_1$, $R_2$ du composé de formule (I) sont obtenus soit en utilisant comme produit de départ le composé de formule (II) convenablement substitué, soit par introduction du substituant choisi à la fin de la synthèse selon les techniques classiques de substitution du noyau aromatique.

Les composés de formule (I) comportant en A une double liaison peuvent subir, le cas échéant, une réduction pour conduire aux composés correspondants hydrogénés de formule (I).

La présente invention a également pour objet les compositions pharmaceutiques renfermant comme principe actif au moins un composé de formule (I) seul ou en combinaison avec un ou plusieurs excipients ou véhicules inertes non toxiques.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les gélules, les tablettes, les suppositoires, les crèmes, pommades, gels dermiques, etc...

La posologie utile varie selon l'âge et le poids du patient, la nature et la sévérité de l'affection ainsi que la voie d'administration. Celle-ci peut être orale, nasale, topique, rectale ou parentérale. D'une manière générale, la posologie unitaire s'échelonne entre 1 mg et 1000 mg pour un traitement en 1 à 3 prises par 24 heures.

Les exemples suivants illustrent l'invention et ne la limitent en aucune façon.
Les préparations suivantes conduisent à des intermédiaires de synthèse utiles dans la préparation des composés de l'invention.

Les structures des composés de l'invention ont été confirmées par les techniques spectroscopiques usuelles (résonance magnétique nucléaire, infrarouge, spectrométrie de masse, ...).

**Préparation A : Diéthylester de l'acide 4-aminophénylphosphonique**

***Stade A: 4-Bromophényldibenzylamine***

A une solution de *p*-bromo aniline (50 g, 0,290 mol) dans l'acétonitrile (500 ml) à 5°C on ajoute la pyridine (56,3 ml, 0,698 mol) puis goutte à goutte le bromure de benzyle (84 ml, 0,698 mol). On agite une nuit à température ambiante puis 1 heure à reflux. L'acétonitrile est évaporé et le résidu est repris dans le dichlorométhane. La phase organique est lavée avec HCl 1N puis NaCl saturé, séchée sur sulfate de sodium et évaporée pour donner 68,5 g d'un mélange de mono- et di-benzylé (50/50). Le mélange est séparé par chromatographie sur silice (cyclohexane/acétate d'éthyle : 70/30) et conduit au produit attendu.
*Point de fusion : 123°C*

***Stade B: Diéthylester de l'acide 4-dibenzylaminophénylphosphonique***

On chauffe au reflux et sous courant d'azote une solution de 50 ml de THF et 10 ml de DMF contenant 27 g du composé décrit au stade A, 23 ml (178 mmol) de diéthylphosphite, 25 ml (179 mmol) de triéthylamine et 3,5 g (3,5 % molaire environ) de Pd(0)tétrakistriphénylphosphine. Après 1 h de reflux on rajoute 1,6 g de Pd(0)tétrakistriphénylphosphine et on poursuit le reflux 8 h. .On rajoute 10 ml de THF, 10ml de triéthylamine, et 1,5 g de Pd(0)tétrakistriphénylphosphine. On poursuit le chauffage au reflux pendant 24 h. On filtre un précipité jaune (catalyseur) du milieu réactionnel, on concentre le filtrat que l'on reprend dans l'acétate d'éthyle. On lave la phase organique avec HCl 1N puis NaCl saturé et on sèche sur sulfate de magnésium. Le brut réactionnel (38 g) est chromatographié sur silice en éluant avec un mélange 50/50 cyclohexane/acétate d'éthyle puis acétate d'éthyle 100 % et conduit au produit attendu.

***Stade C: Diéthylester de l'acide 4-aminophénylphosphonique***

Le composé décrit au stade B précédent (10 g) est dissout dans 400 ml d' éthanol et on hydrogène en présence de 2 g de Pd 10%/C humide sous 3 bars dans un appareil de Parr en chauffant à 60°C pendant 24 h. Le catalyseur est filtré et le filtrat évaporé à sec pour conduire à 7 g d' acide amino-4 phényl phosphonique diéthylester pur.
*Point de fusion : 112-116 °C*

| *Microanalyse élémentaire :* | | | |
|---|---|---|---|
| | *C %* | *H %* | *N %* |
| *calculé* | *52,40* | *7,04* | *6,11* |

(suite)

| Microanalyse élémentaire : | | | |
|---|---|---|---|
| | C % | H % | N % |
| trouvé | 52,61 | 7,06 | 5,97 |

**Préparation B : Diéthylester de l'acide 3-aminophénylphosphonique**

Le produit attendu est obtenu selon le procédé décrit dans la préparation A en utilisant comme produit de départ la 3-bromoaniline.

**Préparation C : Diéthylester de l'acide 3-aminophényl-1,5-diphosphonique**

Le produit attendu est obtenu selon le procédé décrit dans la préparation A en utilisant comme produit de départ la 3,5-dibromoaniline décrite dans J. Org. Chem., 24, 595-598, 1959.

**Préparation des chlorures de diacides**:

Les chlorures de diacides ont été préparés à partir des acides dicarboxyliques correspondants par traitement avec du chlorure de thionyle. Les acides dicarboxyliques sont soit des produits usuels commercialement, soit sont obtenus comme indiqué dans les préparations suivantes :

**Préparation D : Acide N-éthylcarbazole-3,6-dicarboxylique**

L'acide N-éthylcarbazole-3,6-dicarboxylique a été préparé selon le procédé décrit dans le brevet US 4,599,399.

**Préparation E : Acide N-éthylcarbazole-3,6-diacrylique**

***Stade A: 3,6-Diformyl-N-éthylcarbazole***

A une suspension d'acide N-éthyl carbazole-3,6-dicarboxylique (5,7 g, 20,12 mmol) dans 160 ml de THF anhydre refroidit à 0°C on ajoute portion par portion du $LiAlH_4$ (2,15 g, 56,3 mmol). La suspension est agitée 1 h 30 à température ambiante puis 1 heure au reflux. Après refroidissement à 0°C, on hydrolyse l'excès d'hydrure avec un peu d'eau et on filtre les sels. Le filtrat est évaporé à sec et séché sous vide pour donner un solide jaune. Celui-ci est mis en suspension dans du dichlorométhane en présence d'oxyde de manganèse activé (4 g, 15,16 mmol) et on agite une nuit à température ambiante. On ajoute 4 g d'oxyde de manganèse activé et on agite 24 h supplémentaires. La suspension est filtrée et le filtrat est évaporé sous vide pour donner le produit attendu.
*Point de fusion : 170-174 °C*

| Microanalyse élémentaire : | | | |
|---|---|---|---|
| | C % | H % | N % |
| calculé | 76,48 | 5,21 | 5,57 |
| trouvé | 76,59 | 5,42 | 5,60 |

***Stade B: Diéthylester de l'acide N-éthylcarbazole-3,6-diacrylique***

A une suspension du composé obtenu au stade précédent (1,55g, 6,17 mmol) dans 30 ml de méthanol, on ajoute du méthanolate de sodium 5,25 M (2,47 ml, 13 mmol) puis goutte à goutte du triméthylphosphonoacétate (2,0 ml, 12,35 mmol). La suspension est agitée 1 h à température ambiante. Le solvant est évaporé sous vide et le résidu est repris dans l'eau. Le précipité blanc qui s'est formé est filtré, rincé avec un peu d'eau puis d'acétonitrile et séché sous vide pour donner le produit attendu.
*Point de fusion : 164-168° C*

| *Microanalyse élémentaire :* | | | |
|---|---|---|---|
| | *C %* | *H %* | *N %* |
| *calculé* | *72,71* | *5,82* | *3,85* |
| *trouvé* | *72,15* | *5,65* | *3,78* |

***Stade C: Acide N-éthylcarbazole-3,6-diacrylique***

On chauffe à 100°C pendant 3 h le diester obtenu au stade précédent dans 30 ml de NaOH 1N. On laisse revenir à température ambiante et on filtre quelques insolubles. Le filtrat est acidifié avec HCl 3N et on filtre le précipité.
*Point de fusion : 290-300° C*

| *Microanalyse élémentaire :* | | | |
|---|---|---|---|
| | *C %* | *H %* | *N %* |
| *calculé* | *71,63* | *5,11* | *4,18* |
| *trouvé* | *71,54* | *5,20* | *3,95* |

**Préparation F : Acide 9,10-dihydroanthracène-2,6-dicarboxylique**

A une suspension de chlorure d'aluminium (9,24 g, 69,3 mmol) dans 50 ml de dichlorométhane refroidit à -10°C on ajoute goutte à goutte et sous agitation une solution de chlorure d'oxalyle (6,07 ml, 69,3 mmol) dans 50 ml de dichlorométhane puis une solution de 9,10-dihydroanthracène dans 50 ml de dichlorométhane. La suspension est agitée 4 h à -10°C. L' excés de chlorure d'aluminium est hydrolysé par addition goutte à goutte de HCl 1N et la phase organique est rapidement lavée avec de l'eau puis une solution de NaCl saturée et sèchée sur MgSO4. Après évaporation à sec, le résidu est repris dans du chlorure de thionyle (50 ml). La solution est agitée une nuit au reflux. Après évaporation à sec, le résidu est repris dans 100 ml de méthanol anhydre. La solution est agitée une nuit au reflux puis concentrée à 50 ml. Le précipité est filtré pour donner l'acide 9,10-dihydroanthracène 2,6-dicarboxylique diméthylester et l'isomère de position 3,6. Ce mélange est saponifié à 60°C dans 25 ml de NaOH 1N. Après acidification par addition de HCl 1N, le précipité est filtré pour donner le produit attendu.

**Préparation G : Acide benzène-1,4-diacrylique**

***Stade A: Diméthylester de l'acide benzène-1,4-diacrylique***

A une solution de terephtalaldéhyde (9,0 g, 67,1 mmol) dans 75 ml de méthanol et 27 ml de méthanolate de sodium 5,25M (141 mmol), on ajoute goutte à goutte du triméthylphosphonoacétate (22 ml, 135 mmol). Après un léger échauffement, un précipité se forme. La suspension est agitée 1 heure à température ambiante. Le solvant est évaporé sous vide et le résidu est repris dans l'eau. Le précipité blanc est filtré, Rincé avec un peu d'eau puis d'éther et séché sous vide pour donner le produit attendu.
*Point de fusion : 163-167° C*

| *Microanalyse élémentaire :* | | |
|---|---|---|
| | *C %* | *H %* |
| *calculé* | *68,28* | *5,73* |
| *trouvé* | *68,00* | *5,65* |

***Stade B: Acide benzène-1,4-diacrylique***

Une suspension de l'ester précédent (7,0 g, 28,4 mmol) dans 200 ml de NaOH 1N est agitée au reflux pendant une nuit. Après refroidissement à température ambiante, le milieu réactionnel est acidifié avec HCl 6N et le précipité blanc est filtré pour donner le produit attendu.
*Point de fusion : > 300° C*

| *Microanalyse élémentaire :* | | |
|---|---|---|
| | *C %* | *H %* |
| *calculé* | *68,28* | *5,73* |
| *trouvé* | *68,00* | *5,68* |

**Préparation H : Acide benzène-1,3-diacrylique**

Le produit attendu est obtenu selon le procédé décrit dans Chem. Ber., 92, 2532-2542, 1959.

**Préparation I : Acide diphénylméthane-4,4'-diacrylique**

Le composé est préparé selon le procédé décrit dans la préparation G en utilisant la diphénylméthane-4,4'-diformyl.

**Préparation J : Acide diphénylméthane-3,3'-dicarboxylique**

Le produit attendu est obtenu àselon le procédé décrit dans Chem. Ber., 27, 2321-2326, 1894.

**Préparation K : Acide dibenzosubérane-3,7-diacrylique**

Le composé est préparé à partir du dibenzosubérane par réaction de Friedel et Crafts.selon le procédé décrit dans la préparation F puis par réaction d'homologation selon le procédé décrit dans la préparation E.

**Préparation L : Acide cyclohexane-1,4-*trans*-dipropionique**

***Stade A: trans-1,4-Diformyl-cyclohexane***

A une suspension de *trans*-1,4-bis(hydroxyméthyl)-cyclohexane (13,8g, 96 mmol) dans 300 ml de dichlorométhane sont ajoutés en plusieurs fois 41,2 g (191 mmol) de chlorochromate de pyridinium. Après 1 h 30 d'agitation à température ambiante on rajoute 4 g de chlorochromate de pyridinium supplémentaires et on poursuit l'agitation 15 min. Le milieu réactionnel est filtré sur célite et le filtrat est passé sur silice. Après évaporation on obtient une huile qui est utilisée au stade B sans plus de purification

***Stade B: acide cyclohexane-1,4-trans-dipropionique***

Par réaction de wittig selon le stade A de la préparation G suivie d'une hydrogénation catalytique puis d'une saponification comme décrit au stade B de la préparation G.
*Point de fusion: 228-232 °C*

| *Microanalyse élémentaire:* | | |
|---|---|---|
| | *C%* | *H%* |
| *calculé* | *63,14* | *8,83* |
| *trouvé* | *62,42* | *8,51* |

**Préparation M : Acide furane-2,5-diacrylique**

Le composé est préparé à partir du 2,5-diméthanol furane selon le procédé décrit dans la préparation E.
*Point de fusion: 292-296° C*

**Préparation N : Acide thiophène-2,5-diacrylique**

Préparé à partir de l'acide thiophène-2,5-dicarboxylique selon le procédé décrit dans la préparation E.
*Point de fusion: > 300 °C*

| Microanalyse élémentaire: | | | |
|---|---|---|---|
| | C% | H% | S% |
| calculé | 53,56 | 3,60 | 14,30 |
| trouvé | 53,54 | 3,90 | 13,91 |

**Préparation O : Acide naphtalène-2,6-diacrylique**

Préparé à partir de l'acide naphtalène-2,6-dicarboxylique selon le procédé décrit dans la préparation E.
*Point de fusion: > 260 °C*

**Préparation P : Acide fluorène-2,7-diacrylique**

Le composé est préparé à partir du fluorène par réaction de Friedel et Crafts selon le procédé décrit dans la préparation F puis par réaction d'homologation selon le procédé décrit dans la préparation E.
*Point de fusion: > 300 °C*

| Microanalyse élémentaire: | | |
|---|---|---|
| | C% | H% |
| calculé | 74,50 | 4,61 |
| trouvé | 74,80 | 4,44 |

**Préparation Q : Acide naphtalène-1,4-diacrylique (mélange cis-trans 17/23)**

Préparé à partir de l'acide naphtalène-1,4-dicarboxylique selon le procédé décrit dans la préparation E. On obtient de l'acide naphtalène-1,4-diacrylique sous forme d' un mélange cis-trans (17/23).

**Préparation R : Acide cyclohexane-1,4-trans-diacrylique**

Préparé selon la préparation L en excluant l'étape d'hydrogénation catalytique.
*Point de fusion: 299-303 °C*

| Microanalyse élémentaire: | | |
|---|---|---|
| | C% | H% |
| calculé | 64,27 | 7,19 |
| trouvé | 63,88 | 6,89 |

**Préparation S : Diéthylester de l'acide 4-amino styryle phosphonique**

***Stade A: Diéthylester de l'acide 4-nitro styryle phosphonique***

A une solution de 4-nitrobenzaldéhyde (10 g, 66,2 mmol) dans 200 ml de méthanol, on ajoute l'ethylate de sodium (4,95 g, 72,8 mmol) puis goutte à goutte le tétraéthyl méthylène diphosphonate (21 g, 72,8 mmol). Aprés 2 h d'agitation à température ambiante, on rajoute 0,43 g d'éthylate de sodium et 2 g de tétraéthyl méthylène diphosphonate. Aprés 15 min d'agitation à température ambiante, l'éthanol est évaporé sous vide et le résidu est bipartitionné dans un mélange eau / acétate d'éthyle. La phase organique est lavée à l'eau puis séchée ($MgSO_4$) et évaporée sous vide pour donner le produit attendu.
*Point de fusion: 95-98 °C*

| Microanalyse élémentaire: | | | |
|---|---|---|---|
| | C% | H% | N% |
| calculé | 50,53 | 5,65 | 4,91 |

(suite)

| Microanalyse élémentaire: | | | |
|---|---|---|---|
| | C% | H% | N% |
| trouvé | 50,62 | 5,71 | 4,82 |

***Stade B: Diéthylester de l'acide 4-amino styryle phosphonique***

Une suspension contenant du diéthylester de l'acide 4-nitrostyryle phosphonique (3,0 g, 10,5 mmol), du fer en poudre (5,58 g, 100 mmol) et du chlorure d'ammonium (5,58 g, 104 mmol) est chauffée au reflux pendant 45 minutes. Le mileu réactionnel est filtré sur célite. Le filtrat est concentré sous vide et est bipartionné dans un mélange eau / dichlorométhane. La phase organique est lavée à l'eau puis séchée ($MgSO_4$) et évaporée sous vide pour donner le produit attendu qui cristallise peu à peu.
*Point de fusion: 81 °C*

| Microanalyse élémentaire: | | | |
|---|---|---|---|
| | C% | H% | N% |
| calculé | 56,47 | 7,11 | 5,49 |
| trouvé | 56,35 | 7,00 | 5,42 |

**Préparation T : Acide benzène-1,4-di-(1,3-*trans,trans*-butadiénylène) dicarboxylique**

***Stade A: Diéthylester de acide benzène-1,4-di-(1,3-trans trans butadiénylène) dicarboxylique***

A une solution de triéthyl 4 phosphonocrotonate (4,3 ml, 19,4 mmol) dans 10 ml de THF à -70 °C, on ajoute 8,2 ml (16,4 mmol) de butyl lithium 2M dans le pentane. On agite 15 min à -70 °C et on ajoute goutte à goutte 1 g (7,46 mmol) de terephtalaldéhyde en solution dans 15 ml de THF. On laisse revenir à température ambiante en environ 3 heures et on ajoute 75 ml (goutte à goutte au départ) d'une solution aqueuse saturée de NaCl. La réaction est extraite à l'acétate d'éthyle, les phases organiques sont rassemblées, lavées avec une solution de NaCl saturée, séchées sur sulfate de magnésium. Après évaporation sous vide , on obtient le produit attendu sous forme d'un solide orange.
*Point de fusion: 129-135 °C*

| Microanalyse élémentaire: | | |
|---|---|---|
| | C% | H% |
| calculé | 73,60 | 6,79 |
| trouvé | 73,43 | 6,83 |

***Stade B: Acide benzène-1,4-di-(1,3-trans trans butadiénylène) dicarboxylique***

*Point* de fusion: *> 300° C*

| Microanalyse élémentaire: | | |
|---|---|---|
| | C% | H% |
| calculé | 71,10 | 5,22 |
| trouvé | 70,82 | 5,176 |

**Préparation U : Diéthylester de l'acide [2-(4-amino phényl) éthyl] phosphonique**

Obtenu aprés hydrogénation catalytique du diéthylester de l'acide 4-nitro styryle phosphonique (stade A de la préparation S).
*Point de fusion: Huile*

| Microanalyse élémentaire: | | | |
|---|---|---|---|
| | C% | H% | N% |
| calculé | 56,02 | 7,84 | 5,44 |
| trouvé | 56,23 | 8,07 | 5,32 |

**Préparation V : Acide anthracène-9,10-diacrylique**

Préparé par réaction de wittig (conditions décrites dans la préparation G) sur le 9,10-dicarboxaldéhyde anthracène (décrit par K.C. MURDOCK et col., J. Med. Chem., 1982, 25, 505-519).
*Point de fusion: > 260 ° C*

**Préparation W : Diéthylester de l'acide 2-amino phényl phosphonique**

Le produit attendu est obtenu selon le procédé décrit dans la préparation A en utilisant comme produit de départ la 2-bromoaniline.

***Exemple 1:* *Acide 4,4'-[1,8-octanediylbis(carbonylamino)]bis(phénylphosphonique), sel tétrasodique***

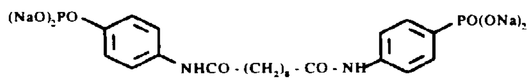

***Stade A: Diéthylester de l'acide 4,4'-[1,8-octanediylbis(carbonylamino)]bis (phénylphosphonique)***

A une solution du composé décrit dans la préparation A (1,87 g, 8,16 mmoles) dans l'acétonitrile (15 ml) on ajoute la pyridine (0,988 ml, 12,24 mmoles) puis une solution de chlorure de sebacoyle (1,12 ml, 5,23 mmoles) dans l'acétonitrile (5 ml). On agite une nuit à température ambiante. Le précipité blanc qui s'est formé est filtré, rincé avec un peu d'acétonitrile et séché sous vide.
*Point de fusion : 172° C*

| Microanalyse élémentaire : | | | |
|---|---|---|---|
| | C % | H % | N % |
| calculé | 57,69 | 7,42 | 4,48 |
| trouvé | 57,53 | 7,24 | 4,46 |

***Stade B: Acide 4,4'-[1,8-octanediylbis(carbonylamino)]bis(phénylphosphonique)***

Une solution de l'ester obtenu au stade A (2,0 g, 3,2 mmol) dans un mélange de bromotriméthylsilane (15 ml) et d'acétonitrile (5 ml) est agitée au reflux pendant 2 h. Après évaporation sous vide, le résidu est agité dans du méthanol . Un précipité se forme, il est filtré pour conduire à l'acide attendu.
*Point de fusion : > 260° C*

| Microanalyse élémentaire : | | | |
|---|---|---|---|
| | C % | H % | N % |
| calculé | 51,57 | 5,90 | 5,47 |
| trouvé | 51,36 | 6,09 | 5,36 |

***Stade C: Acide 4,4'-[1,8-octanediylbis(carbonylamino)]bis(phénylphosphonique), sel tétrasodique***

L' acide obtenu au stade B (1,60 g, 3,12 mmol) est dissous dans 12,49 ml de NaOH 1N et la solution est lyophilysée.
*Point de fusion : > 300° C*

| *Microanalyse élémentaire :* | | | |
|---|---|---|---|
| | *C %* | *H %* | *N %* |
| *caculé* | *44,01* | *4,37* | *4,67* |
| *trouvé* | *44,07* | *4,58* | *4,62* |

Les composés décrits dans les exemples suivants sont obtenus selon le procédé décrit dans l'exemple 1 à partir des produits de départ correspondants.

***Exemple 2**: Acide 4,4'-[1,6-hexanediylbis(carbonylamino)]bis(phénylphosphonique)*

Produits de départ : Chlorure de subéroyl et composé décrit dans la préparation A.

***Stade A : Diéthylester de l'acide 4,4'-[1,6-hexanediylbis(carbonylamino)]bis (phénylphosphonique)***

*Point de fusion : 179 ° C*

| *Microanalyse élémentaire :* | | | |
|---|---|---|---|
| | *C %* | *H %* | *N %* |
| *calculé* | *56,37* | *7,10* | *4,70* |
| *trouvé* | *56,62* | *7,05* | *4,63* |

***Stade B : Acide 4,4'-[1,6-hexanediylbis(carbonylamino)]bis(phénylphosphonique)***

*Point de fusion : >250° C*

| *Microanalyse élémentaire :* | | | |
|---|---|---|---|
| | *C %* | *H %* | *N %* |
| *calculé* | *49,80* | *5,02* | *5,81* |
| *trouvé* | *49,82* | *5,44* | *5,74* |

***Exemple 3**: Acide 4,4'-[1,7-heptanediylbis(carbonylamino)]bis(phénylphosphonique), sel tétrasodique*

Produits de départ: Chlorure d'azélaoyle et composé décrit dans la préparation A.

***Stade A: Diéthylester de l'acide 4,4'-[1,7-heptanediylbis(carbonylamino)]bis (phénylphosphonique)***

*Point de fusion : 121° C*

| *Microanalyse élémentaire :* | | | |
|---|---|---|---|
| | *C %* | *H %* | *N %* |
| *calculé* | *57,04* | *7,26* | *4,59* |
| *trouvé* | *56,51* | *7,20* | *4,68* |

***Stade B: Acide 4,4'-[1,7-heptanediylbis(carbonylamino)]bis(phénylphosphonique)***

*Point de fusion : > 250° C*

| *Microanalyse élémentaire :* | | | |
|---|---|---|---|
| | *C %* | *H %* | *N %* |
| *calculé* | *50,61* | *5,66* | *5,62* |

(suite)

| *Microanalyse élémentaire :* | | | |
|---|---|---|---|
| | *C %* | *H %* | *N %* |
| *trouvé* | *50,31* | *5,83* | *5,49* |

***Stade C: Acide 4,4'-[1,7-heptanediylbis(carbonylamino)]bis(phénylphosphonique), sel tétrasodique***

*Point de fusion : > 250° C*

| *Microanalyse élémentaire :* | | | |
|---|---|---|---|
| | *C %* | *H %* | *N %* |
| *calculé* | *43,02* | *4,13* | *4,78* |
| *trouvé* | *42,96* | *4,12* | *4,77* |

***Exemple 4:** Acide 4,4'-[1,9-nonanediylbis(carbonylamino)]bis(phénylphosphonique)*

Produits de départ : Chlorure de l'acide 1,11-undécanedioique et composé décrit dans la préparation A.

***Stade A: Diéthylester de l'acide 4,4'-[1,9-nonanediylbis(carbonylamino)]bis (phénylphosphonique)***

***Stade B: Acide 4,4'-[1,9-nonanediylbis(carbonylamino)]bis(phénylphosphonique***

*Point de fusion : > 250° C*

| *Microanalyse élémentaire :* | | | |
|---|---|---|---|
| | *C %* | *H %* | *N %* |
| *calculé* | *52,47* | *6,13* | *5,32* |
| *trouvé* | *51,96* | *6,07* | *5,16* |

***Exemple 5: Acide 4,4'-[1,10-décanediylbis(carbonylamino)]bis(phénylphosphonique), sel tétrasodique***

Produits de départ : Chlorure de l'acide 1,12-dodécanedioique et composé décrit dans la préparation A.

***Stade A: Diéthylester de l'acide 4,4'-[1,10-décanediylbis(carbonylamino)]bis (phénylphosphonique)***

*Point* de fusion : *137° C*

***Stade B: Acide 4,4'-[1,10-décanediylbis(carbonylamino)]bis(phénylphosphonique)***

*Point de fusion : > 260° C*

***Stade C: Acide 4,4'-[1,10-décanediylbis(carbonylamino)]bis(phénylphosphonique), sel tétrasodique***

*Point de fusion : > 260° C*

| *Microanalyse élémentaire :* | | | |
|---|---|---|---|
| | *C %* | *H %* | *N %* |
| *calculé* | *45,87* | *4,81* | *4,46* |
| *trouvé* | *45,83* | *4,85* | *4,17* |

***Exemple 6*: Acide 4,4'-[1,11-undécanediylbis(carbonylamino)]bis(phénylphosphonique)**

Produits de départ : Chlorure de l'acide 1,13-tridécanedioique et composé décrit dans la préparation A.

***Stade A: Diéthylester de l'acide 4,4'-[1,11-undécanediylbis(carbonylamino)]bis (phénylphosphonique)***

***Stade B: Acide 4,4'-[1,11-undécanediylbis(carbonylamino)]bis(phénylphosphonique)***

*Point de fusion : >250° C*

| *Microanalyse élémentaire :* | | | |
|---|---|---|---|
| | *C %* | *H %* | *N %* |
| *calculé* | *54,15* | *6,54* | *5,05* |
| *trouvé* | *54,33* | *6,51* | *4,88* |

***Exemple 7*: Acide 4,4'-[1,12-dodécanediylbis(carbonylamino)]bis(phénylphosphonique)**

Produits de départ : Chlorure de l'acide 1,14-tetradécanedioique et composé décrit dans la préparation A.

***Stade A: Diéthylester de l'acide 4,4'-[1,12-dodécanediylbis(carbonylamino)]bis (phénylphosphonique)***

*Point de fusion : 128° C*

| *Microanalyse élémentaire :* | | | |
|---|---|---|---|
| | *C %* | *H %* | *N %* |
| *calculé* | *59,99* | *8,00* | *4,11* |
| *trouvé* | *59,74* | *8,07* | *4,16* |

***Stade B: Acide 4,4'[1,12-dodécanediylbis(carbonylamino)]bis(phénylphosphonique)***

*Point* de fusion : *> 250° C*

| *Microanalyse élémentaire :* | | | |
|---|---|---|---|
| | *C %* | *H %* | *N %* |
| *calculé* | *54,93* | *6,74* | *4,93* |
| *trouvé* | *54,88* | *6,90* | *4,92* |

***Exemple 8*: Acide 4,4'-[1,13-tridécanediylbis(carbonylamino)]bis(phénylphosphonique)**

Produits de départ : Chlorure de l'acide 1,15-pentadécanedioique et composé décrit dans la préparation A.

***Stade A: Diéthylester de l'acide 4,4'-[1,13-tridécanediylbis(carbonylamino)]bis (phénylphosphonique)***

***Stade B: Acide 4,4'-[1,13-tridécanediylbis(carbonylamino)]bis(phénylphosphonique)***

*Point de fusion : > 250° C*

| *Microanalyse élémentaire :* | | | |
|---|---|---|---|
| | *C %* | *H %* | *N %* |
| *calculé* | *55,67* | *6,92* | *4,81* |
| *trouvé* | *55,45* | *6,84* | *4,52* |

***Exemple 9*: Acide 4,4'-[1,14-tétradécanediylbis(carbonylamino)]bis(phényiphosphonique)**

Produits de départ : Chlorure de l'acide 1,16-hexadécanedioique et composé décrit dans la préparation A.

***Stade A: Diéthylester de l'acide 4,4'-[1,14-tétradécanediylbis(carbonylamino)]bis (phénylphosphonique)***

*Point de fusion : 130° C*

| *Microanalyse élémentaire :* | | | |
|---|---|---|---|
| | *C %* | *H %* | *N %* |
| *calculé* | *61,00* | *8,25* | *3,95* |
| *trouvé* | *61,74* | *8,30* | *3,85* |

***Stade B: Acide 4,4'-[1,14-tétradécanediylbis(carbonylamino)]bis(phénylphosphonique)***

*Point de fusion : > 250° C*

| *Microanalyse élémentaire :* | | | |
|---|---|---|---|
| | *C %* | *H %* | *N %* |
| *calculé* | *56,37* | *7,10* | *4,70* |
| *trouvé* | *56,37* | *7,06* | *4,49* |

***Exemple 10*: Acide 4,4'-[1,3-phényldiylbis(acétamino)]bis(phénylphosphonique), sel tétrasodique**

Produits de départ : Chlorure de l'acide benzène-1,3-diacétique et composé décrit dans la préparation A.

***Stade A: Diéthylester de l'acide 4,4'-[1,3-phényldiylbis(acétamino)]bis (phénylphosphonique)***

*Point de fusion : 162° C*

| *Microanalyse élémentaire :* | | | |
|---|---|---|---|
| | *C %* | *H %* | *N %* |
| *calculé* | *58,44* | *6,21* | *4,54* |
| *trouvé* | *58,05* | *6,18* | *4,27* |

***Stade B: Acide 4,4'-[1,3-phényldiylbis(acétamino)]bis(phénylphosphonique)***

*Point de fusion : > 250° C*

| *Microanalyse élémentaire :* | | | |
|---|---|---|---|
| | *C %* | *H %* | *N %* |
| *calculé* | *57,25* | *4,77* | *4,95* |
| *trouvé* | *57,79* | *4,19* | *5,05* |

***Stade C: Acide 4,4'-[1,3-phényldiylbis(acétamino)]bis(phénylphosphonique), sel tétrasodique***

*Point* de fusion : > *250° C*

| *Microanalyse élémentaire :* | | | |
|---|---|---|---|
| | *C %* | *H %* | *N %* |
| *calculé* | *44,61* | *3,06* | *4,73* |

(suite)

| *Microanalyse élémentaire :* | | | |
|---|---|---|---|
| | *C %* | *H %* | *N %* |
| *trouvé* | *44,38* | *3,17* | *4,02* |

***Exemple 11*: Acide 4,4'-[4,4'-diphénylméthanediylbis(carbonylamino)]bis(phényl phosphonique), sel tétrasodique**

Produits de départ : Chlorure de l'acide diphénylméthane-4,4'-dicarboxylique et composé décrit dans la préparation A.

***Stade A: Diéthylester de l'acide 4,4'-[4,4'-diphénylméthanediylbis(carbonylamino)]bis (phénylphosphonique)***

*Point de fusion : 242-243° C*

| *Microanalyse élémentaire :* | | | |
|---|---|---|---|
| | *C %* | *H %* | *N %* |
| *calculé* | *61,94* | *5,94* | *4,13* |
| *trouvé* | *61,55* | *6,03* | *4,09* |

***Stade B: Acide 4,4'-[4,4'-diphénylméthanediylbis(carbonylamino)]bis (phénylphosphonique)***

*Point de fusion : > 250° C*

| *Microanalyse élémentaire :* | | | |
|---|---|---|---|
| | *C %* | *H %* | *N %* |
| *calculé* | *57,25* | *4,27* | *4,95* |
| *trouvé* | *57,37* | *4,33* | *4,76* |

***Stade C: Acide 4,4'-[4,4'-diphénylméthanediylbis(carbonylamino)]bis (phénylphosphonique), sel tétrasodique***

*Point de fusion : > 250° C*

| *Microanalyse élémentaire :* | | | |
|---|---|---|---|
| | *C %* | *H %* | *N %* |
| *calculé* | *49,56* | *3,08* | *4,28* |
| *trouvé* | *49,96* | *3,37* | *4,24* |

***Exemple 12*: Acide 4,4'-[4,4'-(biphényldiylbis(sulfonylamino)]bis(phénylphosphonique), sel tétrasodique**

Produits de départ : Chlorure de l'acide biphényl-4,4'-dicarboxylique et composé décrit dans la préparation A.

***Stade A: Diéthylester de l'acide 4,4'-[4,4'-biphényldiylbis(sulfonylamino)]bis (phénylphosphonique)***

*Point de fusion : >260° C*

| *Microanalyse élémentaire :* | | | | |
|---|---|---|---|---|
| | *C %* | *H %* | *N %* | *S %* |
| *calculé* | *52,17* | *5,20* | *3,80* | *8,70* |
| *trouvé* | *52,22* | *5,10* | *3,74* | *9,07* |

***Stade B: Acide 4,4'-[4,4'-biphényldiylbis(sulfonylamino)]bis(phénylphosphonique)***

*Point de fusion : > 260° C*

***Stade C: Acide 4,4'-[4,4'-biphényldiylbis(sulfonylamino)]bis(phénylphosphonique), sel tétrasodique***

*Point de fusion : > 280° C*

| *Microanalyse élémentaire :* | | | | |
|---|---|---|---|---|
| | *C %* | *H %* | *N %* | *S %* |
| *calculé* | *40,46* | *2,55* | *3,93* | *9,00* |
| *trouvé* | *38,81* | *2,80* | *3,65* | *8,15* |

***Exemple 13: Acide 4,4'-[4,4'-diphénylsulfonediylbis(carbonylamino)]bis(phényl phosphonique), sel tétrasodique***

Produits de départ : Chlorure de l'acide diphénylsulfone-4,4'-dicarboxylique et composé décrit dans la préparation A.

***Stade A: Diéthylester de l'acide 4,4'-[4,4'-diphénylsulfonediylbis(carbonylamino)]bis (phénylphosphonique)***

*Point* de fusion : *210° C*

| *Microanalyse élémentaire :* | | | | |
|---|---|---|---|---|
| | *C %* | *H %* | *N %* | *S %* |
| *calculé* | *56,04* | *5,26* | *3,84* | *4,40* |
| *trouvé* | *56,83* | *5,38* | *3,89* | *4,25* |

***Stade B: Acide 4,4'-[4,4'-diphénylsulfonediylbis(carbonylamino)]bis (phénylphosphonique)***

*Point de fusion : > 260° C*

| *Microanalyse élémentaire :* | | | | |
|---|---|---|---|---|
| | *C %* | *H %* | *N %* | *S %* |
| *calculé* | *50,66* | *3,60* | *4,54* | *5,20* |
| *trouvé* | *51,06* | *3,78* | *4,61* | *5,15* |

***Stade C: Acide 4,4'-[4,4'-diphénylsulfonediylbis(carbonylamino)]bis(phényl phosphonique), sel tétrasodique***

*Point de fusion : > 250° C*

| *Microanalyse élémentaire :* | | | | |
|---|---|---|---|---|
| | *C %* | *H %* | *N %* | *S %* |
| *calculé* | *44,33* | *2,58* | *3,98* | *4,55* |
| *trouvé* | *44,28* | *2,14* | *4,02* | *4,58* |

***Exemple 14: Acide 4,4'-[N-éthylcarbazole-3,6-diylbis(carbonylamino)]bis(phényl phosphonique)***

Produits de départ : Chlorure de l'acide N-éthylcarbazole-3,6-dicarboxylique décrit dans la préparation D et composé décrit dans la préparation A.

***Stade A: Diéthylester de l'acide 4,4'-[N-éthylcarbazole-3,6-diylbis(carbonylamino)]bis (phénylphosphonique)***

*Point de fusion : 221-225° C*

| *Microanalyse élémentaire :* | | | |
|---|---|---|---|
| | *C %* | *H %* | *N %* |
| *calculé*<br>*trouvé* | *61,27*<br>*60,52* | *5,86*<br>*5,82* | *5,95*<br>*5,95* |

***Stade B: Acide 4,4'-[N-éthylcarbazole-3,6-diylbis(carbonylamino)]bis(phénylphosphonique)***

*Point de fusion : > 300° C*

| *Microanalyse élémentaire :* | | | |
|---|---|---|---|
| | *C %* | *H %* | *N %* |
| *calculé*<br>*trouvé* | *56,67*<br>*56,94* | *4,25*<br>*4,37* | *7,08*<br>*7,03* |

***Exemple 15**: Acide 4,4'-[(9,10-dihydroanthracène)-2,6-diylbis(carbonylamino)]bis(phényl phosphonique)*

Produits de départ : Chlorure de l'acide 9,10-dihydroanthracène-2,6-dicarboxylique décrit dans la préparation F et composé décrit dans la préparation A.

***Stade A: Diéthylester de l'acide 4,4'-[(9,10-dihydroanthracène)-2,6-diylbis (carbonylamino)]bis(phénylphosphonique)***

*Point* de fusion : *> 260° C*

***Stade B: Acide 4,4'-[(9,10-dihydroanthracène)-2,8-diylbis(carbonylamino)]bis(phényl phosphonique)***

*Point* de fusion : *> 260° C*

***Exemple 16**: Acide 4,4'-[4,4'-Biphényldiylbis(carbonylamino)]bis(phénylphosphonique)*

Produits de départ : Chlorure de l'acide 4,4'-biphényldicarboxylique et composé décrit dans la préparation A.

***Stade A: Diéthylester de l'acide 4,4'-[4,4'-biphényldiylbis(carbonylamino)]bis (phénylphosphonique)***

*Point de fusion : > 260° C*

| *Microanalyse élémentaire :* | | | |
|---|---|---|---|
| | *C %* | *H %* | *N %* |
| *calculé*<br>*trouvé* | *61,44*<br>*61,31* | *5,76*<br>*5,86* | *4,21*<br>*4,19* |

***Stade B: Acide 4,4'-[4,4'-Biphényldiylbis(carbonylamino)]bis(phénylphosphonique))***

*Point de fusion : > 260° C*

| *Microanalyse élémentaire :* | | | |
|---|---|---|---|
| | *C %* | *H %* | *N %* |
| *calculé* | *56,53* | *4,01* | *5,07* |

(suite)

| *Microanalyse élémentaire :* | | | |
|---|---|---|---|
| | *C %* | *H %* | *N %* |
| *trouvé* | *56,39* | *3,92* | *5,05* |

***<u>Exemple 17</u>: Acide 4,4'-[3,3'-diphénylméthanediylbis(carbonylamino)]bis(phényl phosphonique)***

<u>Produits de départ</u> : Chlorure de l'acide diphénylméthane-3,3'-dicarboxylique décrit dans la préparation J et composé décrit dans la préparation A.

***Stade A: Diéthylester de l'acide 4,4'-[3,3'-diphénylméthanediylbis(carbonylamino)]bis (phénylphosphonique)***

| *Microanalyse élémentaire :* | | | |
|---|---|---|---|
| | *C %* | *H %* | *N %* |
| *calculé* | *61,94* | *5,94* | *4,13* |
| *trouvé* | *61,72* | *5,75* | *4,00* |

***Stade B: Acide 4,4'-[3,3'-diphénylméthanediylbis(carbonylamino)]bis(phénylphosphonique)***

*Point de fusion : > 260° C*

| *Microanalyse élémentaire :* | | | |
|---|---|---|---|
| | *C %* | *H %* | *N %* |
| *calculé* | *57,25* | *4,27* | *4,95* |
| *trouvé* | *57,37* | *4,33* | *4,76* |

***<u>Exemple 18</u>: Acide 4,4'-[4,4'-*trans*-stilbènediylbis(carbonylamino)]bis(phényl phosphonique), sel tétrasodique***

<u>Produits de départ</u> : Chlorure de l'acide *trans*-stilbène-4,4'-dicarboxylique et composé décrit dans la préparation A.

***Stade A: Diéthylester de l'acide 4,4'-[4,4'-*trans*-stilbènediylbis(carbonylamino)]bis (phénylphosphonique)***

*Point de fusion : > 260° C*

***Stade B: Acide 4,4'-[4,4'-*trans*-stilbènediylbis(carbonylamino)]bis(phényl phosphonique)***

*Point* de fusion : *> 260° C*

| *Microanalyse élémentaire :* | | | |
|---|---|---|---|
| | *C %* | *H %* | *N %* |
| *calculé* | *58,14* | *4,18* | *4,84* |
| *trouvé* | *57,17* | *3,94* | *4,69* |

***Stade C: Acide 4,4'-[4,4'-*trans*-stilbènediylbis(carbonylamino)]bis(phényl phosphonique), sel tétrasodique***

*Point de fusion : > 260° C*

| Microanalyse élémentaire : | | | |
|---|---|---|---|
| | C % | H % | N % |
| calculé | 50,47 | 3,02 | 4,20 |
| trouvé | 50,38 | 3,02 | 4,19 |

***Exemple 19*: Acide 4,4'-[4,4'-(1,2-diphényléthane)diylbis(carbonylamino)]bis(phényl phosphonique), sel tétrasodique**

Produits de départ : Chlorure de l'acide 1,2-diphényléthane-4,4'-dicarboxylique et composé décrit dans la préparation A.

***Stade A: Diéthylester de l'acide 4,4'-[4,4'-(1,2-diphényléthane)diylbis(carbonylamino)] bis(phénylphosphonique)***

*Point de fusion : 244° C*

| Microanalyse élémentaire : | | | |
|---|---|---|---|
| | C % | H % | N % |
| calculé | 62,42 | 6,11 | 4,04 |
| trouvé | 61,85 | 5,95 | 3,88 |

***Stade B: Acide 4,4'-[4,4'-(1,2-diphényléthane)diylbis(carbonylamino)]bis (phénylphosphonique)***

*Point de fusion : > 260° C*

| Microanalyse élémentaire : | | | |
|---|---|---|---|
| | C % | H % | N % |
| calculé | 57,94 | 4,51 | 4,83 |
| trouvé | 57,44 | 4,52 | 4,75 |

***Stade C: Acide 4,4'-[4,4'-(1,2-diphényléthane)diylbis(carbonylamino)]bis(phényl phosphonique), sel tétrasodique***

*Point de fusion : > 260° C*

| Microanalyse élémentaire : | | | |
|---|---|---|---|
| | C % | H % | N % |
| calculé | 50,32 | 3,32 | 4,19 |
| trouvé | 50,13 | 3,31 | 4,17 |

***Exemple 20*: Acide 5,5'-[1,8-octanediylbis(carbonylamino)]bis(phényl-1,3-diphosphonique), sel octasodique**

Produits de départ : Chlorure de sébacoyl et composé décrit dans la préparation C.

***Stade A: Diéthylester de l'acide 5,5'-[1,8-octanediylbis(carbonylamino)]bis(phényl-1,3-diphosphonique)***

***Stade B: Acide 5,5'-[1,8-octanediylbis(carbonylamino)]bis(phényl-1,3-diphosphonique)***

***Stade C: Acide 5,5'-[1,8-octanediylbis(carbonylamino)]bis (phényl-1,3-diphosphonique), sel octasodique***

*Point de fusion : > 250° C*

| *Microanalyse élémentaire :* | | | |
|---|---|---|---|
| | *C %* | *H %* | *N %* |
| *calculé* | *31,15* | *2,85* | *3,30* |
| *trouvé* | *30,94* | *2,91* | *3,33* |

***Exemple 21**: Acide 5,5'-[4,4'-diphénylméthanediylbis(carbonylamino)]bis(phényl-1,3-diphosphonique), sel octasodique*

Produits de départ : Chlorure de l'acide diphénylméthane-4,4'-dicarboxylique et composé décrit dans la préparation C.

***Stade A: Diéthylester de l'acide 5,5'-[4,4'-diphénylméthanediylbis(carbonylamino)]bis (phényl-1,3-diphosphonique)***

*Point de fusion : > 70-72° C*

| *Microanalyse élémentaire :* | | | |
|---|---|---|---|
| | *C %* | *H %* | *N %* |
| *calculé* | *54,32* | *6,15* | *2,95* |
| *trouvé* | *54,92* | *6,69* | *2,80* |

***Stade B: Acide 5,5'-[4,4'-diphénylméthanediylbis(carbonylamino)]bis(phényl-1,3-diphosphonique)***

*Point de fusion : > 250° C*

| *Microanalyse élémentaire :* | | | |
|---|---|---|---|
| | *C %* | *H %* | *N %* |
| *calculé* | *44,64* | *3,61* | *3,86* |
| *trouvé* | *44,04* | *3,72* | *3,65* |

***Stade C: Acide 5,5'-[4,4'-diphénylméthanediylbis(carbonylamino)]bis(phényl-1,3-diphosphonique), sel octasodique***

*Point de fusion : > 250° C*

| *Microanalyse élémentaire :* | | | |
|---|---|---|---|
| | *C %* | *H %* | *N %* |
| *calculé* | *35,94* | *2,01* | *3,10* |
| *trouvé* | *35,56* | *1,79* | *3,13* |

***Exemple 22**: Acide 3,3'-[1,8-octanediylbis(carbonylamino)]bis(phénylphosphonique)*

Produits de départ : Chlorure de sébacoyle et composé décrit dans la préparation B.

***Stade A: Diéthylester de l'acide 3,3'-[1,8-Octanediylbis(carbonylamino)]bis (phénylphosphonique)***

*Point de fusion : 140-150° C*

| *Microanalyse élémentaire :* | | | |
|---|---|---|---|
| | *C %* | *H %* | *N %* |
| *calculé* | *57,69* | *7,42* | *4,48* |
| *trouvé* | *57,68* | *7,55* | *4,24* |

***Stade B: Acide 3,3'-[1,8-octanediylbis(carbonylamino)]bis(phénylphosphonique)***

*Point de fusion : 217-222° C*

| *Microanalyse élémentaire :* | | | |
|---|---|---|---|
| | *C %* | *H %* | *N %* |
| *calculé* | *51,57* | *5,90* | *5,47* |
| *trouvé* | *51,29* | *5,84* | *5,39* |

***Exemple 23: Acide 4,4'-[1,3-divinylènebenzènediylbis(carbonylamino)]bis(phényl phosphonique)***

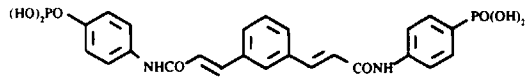

Produits de départ : Composés décrits dans les préparations A et H.

***Stade A: Diéthylester de l'acide 4,4'-[1,3-divinylènebenzènediylbis(carbonylamino)]bis (phénylphosphonique)***

*Point de fusion : 197-204° C*

| *Microanalyse élémentaire :* | | | |
|---|---|---|---|
| | *C %* | *H %* | *N %* |
| *calculé* | *60,00* | *5,98* | *4,37* |
| *trouvé* | *60,12* | *5,87* | *4,22* |

***Stade B: Acide 4,4'-[1,3-divinylènebenzènediylbis(carbonylamino)]bis(phényl phosphonique)***

*Point de fusion : > 300° C*

| *Microanalyse élémentaire :* | | | |
|---|---|---|---|
| | *C %* | *H %* | *N %* |
| *calculé* | *54,55* | *4,20* | *5,30* |
| *trouvé* | *53,70* | *4,21* | *5,07* |

***Exemple 24: Acide 4,4'-[1,4-divinylènebenzènediylbis(carbonylamino)]bis(phényl phosphonique)***

Produits de départ : Composés décrits dans les préparations A et G.

***Stade A: Diéthylester de l'acide 4,4'-[1,4-divinylènelbenzènediylbis(carbonylamino)]bis (phényphosphonique)***

*Point de fusion : 300-305° C*

***Stade B: Acide 4,4'-[1,4-divinylènebenzènediylbis(carbonylamino)]bis(phényl phosphonique)***

*Point de fusion : >300° C*

| *Microanalyse élémentaire :* | | | |
|---|---|---|---|
| | *C %* | *H %* | *N %* |
| *calculé* | *54,55* | *4,20* | *5,30* |
| *trouvé* | *53,29* | *4,34* | *5,08* |

***<u>Exemple 25</u>: Acide 4,4'-[1,4-diéthylènebenzènediylbis(carbonylamino)]bis(phényl phosphonique)***

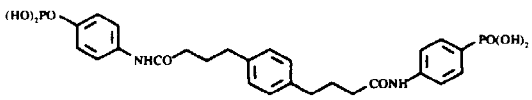

***Stade A: Diéthylester de l'acide 4,4'-[1,4-diéthylènebenzènediylbis(carbonylamino)]bis (phénylphosphonique)***

Le produit décrit au stade A de l'exemple 24 (840 mg, 1.31 mmol) est hydrogéné en présence de 800 mg de formiate d'ammonium et 800 mg de Palladium sur charbon dans 15 ml d'éthanol à reflux pendant 30 min. Le catalyseur est filtré et le filtrat est évaporé à sec pour donner le produit attendu.
*Point de fusion : 264-270° C*

| *Microanalyse élémentaire :* | | | |
|---|---|---|---|
| | *C %* | *H %* | *N %* |
| *calculé* | *59,62* | *6,67* | *4,35* |
| *trouvé* | *58,89* | *6,51* | *4,32* |

***Stade B: Acide 4,4'-[1,4-diéthylènebenzènediylbis(carbonylamino)]bis(phényl phosphonique)***

Le produit attendu est obtenu selon le procédé décrit au stade B de l'exemple 1.
*Point de fusion : > 260° C*

| *Microanalyse élémentaire :* | | | |
|---|---|---|---|
| | *C %* | *H %* | *N %* |
| *calculé* | *54,14* | *4,92* | *5,26* |
| *trouvé* | *53,69* | *4,95* | *5,03* |

***<u>Exemple 26</u>: Acide 4,4'-[4,4'-diéthylènediphénylméthanediylbis(carbonylamino)]bis (phénylphosphonique)***

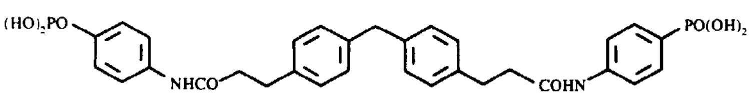

<u>Produits de départ</u> : Composés décrits dans les préparations A et I.

***Stade A: Diéthylester de l'acide 4,4'-[4,4'-(divinylène)diphénylméthanediylbis (carbonylamino)]bis(phénylphosphonique)***

*Point de fusion : 249-254° C*

***Stade B: Diéthylester de l'acide 4,4'-[4,4'-(diéthylène)diphénylméthanediylbis (carbonylamino)]bis(phénylphosphonique)***

Le produit du stade A (560 mg, 0,766 mmol) est hydrogéné en présence de 500 mg de formiate d'ammonium et 500 mg de Palladium sur charbon dans 10 ml d'éthanol à reflux pendant 30 min. Le catalyseur est filtré et le filtrat est évaporé à sec pour donner le produit attendu.
*Point de fusion : 80° C*

***Stade C: Acide 4,4'-[4,4'-(diéthylène)diphénylméthanediylbis(carbonylamino)]bis(phényl phosphonique)***

*Point de fusion : > 300° C*

| *Microanalyse élémentaire :* | | | |
|---|---|---|---|
| | *C %* | *H %* | *N %* |
| *calculé* | *59,81* | *5,18* | *4,50* |
| *trouvé* | *59,44* | *5,33* | *4,33* |

***Exemple 27: Acide 4,4'-[1,8-octanediylbis(carbonylamino)]bis(3-nitrophénylphosphonique)***

***Stade A: Diéthylester de l'acide 4,4'-[1,8-octanediylbis(carbonylamino)]bis (3-nitrophénylphosphonique)***

A 0°C, on prépare une solution contenant 1,8 ml d'acide sulfurique 96 % et 1,2 ml d'acide nitrique 86 %. On prélève 1,1 ml de cette solution que l'on additionne goutte à goutte à une suspension à -5°C du composé décrit au stade A de l'exemple 1 (930 mg, 1,5 mmol) dans 4,6 ml d'acide sulfurique 96 %. Le milieu réactionnel devient peu à peu homogène et après 2 h 30 la solution est versée sur de la glace. On extrait la phase aqueuse plusieurs fois avec du dichlorométhane et le produit est purifié par chromatographie sur silice (dichlorométhane/ méthanol : 97/3)
*Point de fusion : 128° C*

| *Microanalyse élémentaire :* | | | |
|---|---|---|---|
| | *C %* | *H %* | *N %* |
| *calculé* | *50,42* | *6,21* | *7,84* |
| *trouvé* | *50,23* | *6,21* | *7,75* |

***Stade B: Acide 4,4'-[1,8-octanediylbis(carbonylamino)]bis(3-nitrophénylphosphonique)***

*Point de fusion : 227-230° C*

| *Microanalyse élémentaire :* | | | |
|---|---|---|---|
| | *C %* | *H %* | *N %* |
| *calculé* | *43,86* | *4,68* | *9,30* |
| *trouvé* | *43,59* | *5,03* | *8,75* |

***Exemple 28: Acide 4,4'-[3,6-diéthylène N-éthylcarbazole diylbis(carbonylamino)]bis (phénylphosphonique), sel tétrasodique***

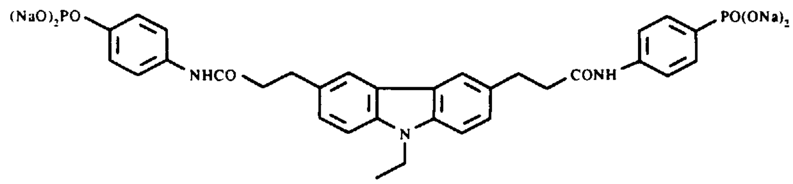

Produits de départ : Composé décrit dans la préparation A et composé obtenu après réduction catalytique du

composé décrit dans la préparation E.

***Stade A: Diéthylester de l'acide 4,4'-[3,6-diéthylène N-éthylcarbazole diylbis (carbonylamino)]bis(phénylphosphonique)***

*Point de fusion : 101-108° C*

| Microanalyse élémentaire : | | | |
|---|---|---|---|
| | C % | H % | N % |
| calculé | 63,07 | 6,48 | 5,52 |
| trouvé | 62,60 | 6,45 | 5,45 |

***Stade B: Acide 4,4'-[3,6-diéthylène N-éthylcarbazole diylbis (carbonylamino)]bis(phénylphosphonique)***

*Point de fusion : 279-285° C*

| Microanalyse élémentaire : | | | |
|---|---|---|---|
| | C % | H % | N % |
| calculé | 59,17 | 5,12 | 6,47 |
| trouvé | 58,96 | 5,15 | 6,28 |

***Stade C: Acide 4,4'-[3,6-diéthylène N-éthylcarbazole diylbis(carbonylamino)]bis (phénylphosphonique), sel tétrasodique***

*Point de fusion : > 300° C*

| Microanalyse élémentaire : | | | |
|---|---|---|---|
| | C % | H % | N % |
| calculé | 52,12 | 3,96 | 5,70 |
| trouvé | 51,78 | 3,71 | 5,53 |

***Exemple 29: Acide 4,4'-[dibenzosubérane-3,7-diylbis(carbonylamino)]]bis (phénylphosphonique), sel tétrasodique***

Produits de départ : Chlorure de l'acide dibenzosubérane-3,7-dicarboxylique (préparé selon le procédé décrit dans la préparation F) et composé décrit dans la préparation A.

***Stade A: Diéthylester de l'acide 4,4'-[dibenzosubérane-3,7-diylbis(carbonylamino)]bis (phénylphosphonique)***

*Point de fusion : 202-205° C*

| Microanalyse élémentaire : | | | |
|---|---|---|---|
| | C % | H % | N % |
| calculé | 63,06 | 6,01 | 3,98 |
| trouvé | 62,91 | 5,85 | 3,84 |

***Stade B: Acide 4,4'-[dibenzosubérane-3,7-diylbis(carbonylamino)]]bis phénylphosphonique)***

*Point de fusion : 292-296° C*

| *Microanalyse élémentaire :* | | | |
|---|---|---|---|
| | *C %* | *H %* | *N %* |
| *calculé* | *58,79* | *4,42* | *4,73* |
| *trouvé* | *58,24* | *4,37* | *4,50* |

***Stade C: Acide 4,4'-[dibenzosubérane-3,7-diylbis(carbonylamino)]]bis phénylphosphonique), sel tétrasodique***

*Point de fusion : > 250° C*

| *Microanalyse élémentaire :* | | | |
|---|---|---|---|
| | *C %* | *H %* | *N %* |
| *calculé* | *51,19* | *3,26* | *4,12* |
| *trouvé* | *51,22* | *3,77* | *4,13* |

***Exemple 30: Acide 4,4'-[3,6-divinylène N-éthylcarbazole diylbis (carbonylamino)]bis(phénylphosphonique), sel tétrasodique***

Produits de départ : Composés décrits dans les préparations A et E.

***Stade A : Diéthylester de l'acide 4,4'-[3,6-divinylène N-éthylcarbazole diylbis (carbonylamino)]bis(phénylphosphonique)***

*Point de fusion : 172-180° C*

***Stade B: Acide 4,4'-[3,6-divinylène N-éthylcarbazole diylbis (carbonylamino)]bis(phénylphosphonique)***

*Point de fusion : > 300° C*

| *Microanalyse élémentaire :* | | | |
|---|---|---|---|
| | *C %* | *H %* | *N %* |
| *calculé* | *59,54* | *4,53* | *6,51* |
| *trouvé* | *59,69* | *4,68* | *6,29* |

***Stade C: Acide 4,4'-[3,6-divinylène N-éthylcarbazole diylbis (carbonylamino)]bis(phénylphosphonique), sel tétrasodique***

*Point de fusion : > 250° C*

| *Microanalyse élémentaire :* | | | |
|---|---|---|---|
| | *C %* | *H %* | *N %* |
| *calculé* | *52,40* | *3,44* | *5,73* |
| *trouvé* | *52,35* | *3,28* | *5,42* |

***Exemple 31: Acide 4,4'-[3,7-divinylène dibenzosubérane diylbis(carbonylamino)] bis(phénylphosphonique), sel tétrasodique***

Produits de départ: Chlorure de l'acide dibenzosubérane-1,3-diacrylique (préparation K) et diéthylester de l'acide 4-aminophénylphosphonique (préparation A).

***Stade A: Diéthylester de l'acide 4,4'-[3,7-divinylène dibenzosubérane diylbis(carbonylamino)]bis(phénylphosphonique)***

*Point de fusion: 179-185 °C*

| *Microanalyse élémentaire:* | | | |
|---|---|---|---|
| | *C%* | *H%* | *N%* |
| *calculé* | *65,07* | *6,13* | *3,70* |
| *trouvé* | *64,53* | *6,03* | *3,58* |

***Stade B: Acide 4,4'-[3,7-divinylène dibenzosubérane diylbis(carbonylamino)] bis(phénylphosphonique)***

*Point de fusion: > 300 °C*

| *Microanalyse élémentaire:* | | | |
|---|---|---|---|
| | *C%* | *H%* | *N%* |
| *calculé* | *61,49* | *4,69* | *4,35* |
| *trouvé* | *61,44* | *4,59* | *4,12* |

***Stade C: Acide 4,4'-[3,7-divinylène dibenzosubérane diylbis(carbonylamino)] bis(phénylphosphonique), sel tétrasodique***

*Point de fusion: > 300 °C*

| *Microanalyse élémentaire:* | | | |
|---|---|---|---|
| | *C%* | *H%* | *N%* |
| *calculé* | *54,11* | *3,58* | *3,82* |
| *trouvé* | *54,30* | *3,31* | *3,63* |

***Exemple 32: Acide 4,4'-[trans 1,4-diéthylènecyclohexanediylbis(carbonylamino)] bis(phénylphosphonique)***

Produits de départ: Chlorure de l'acide cyclohexane-1,4-*trans*-dipropionique (préparation L) et diéthylester de l'acide 4-aminophénylphosphonique (préparation A).

***Stade A: Diéthyl ester de l'acide 4,4'-[trans 1,4-diéthylènecyclohexane diylbis(carbonylamino)]bis(phénylphosphonique)***

*Point de fusion: 255-259 °C*

| *Microanalyse élémentaire:* | | | |
|---|---|---|---|
| | *C%* | *H%* | *N%* |
| *calculé* | *59,07* | *7,44* | *4,31* |
| *trouvé* | *58,75* | *7,27* | *4,48* |

***Stade B: Acide 4,4'-[trans 1,4-diéthylènecyclohexane diylbis(carbonylamino)]bis(phénylphosphonique)***

*Point de fusion:299-305 °C*

| *Microanalyse élémentaire:* | | | |
|---|---|---|---|
| | *C%* | *H%* | *N%* |
| *calculé* | *53,53* | *5,99* | *5,20* |

(suite)

| *Microanalyse élémentaire:* | | | |
|---|---|---|---|
| | *C%* | *H%* | *N%* |
| *trouvé* | *53,16* | *6,09* | *4,93* |

***Exemple 33**: Acide 4,4'-[1,16-hexadécanediylbis(carbonylamino)bis(phénylphosphonique)*

Produits de départ: Chlorure de l'acide 1,18-octadécanedioique et diéthylester de l'acide 4-aminophénylphosphonique (préparation A).

***Stade A: Diéthyl ester de l'acide 4,4'-[1,16-hexadécanediylbis(carbonylamino)] bis(phénylphosphonique)***

*Point de fusion: 104 °C*

| *Microanalyse élémentaire:* | | | |
|---|---|---|---|
| | *C%* | *H%* | *N%* |
| *calculé* | *61,94* | *8,48* | *3,88* |
| *trouvé* | *61,99* | *8,50* | *3,53* |

***Stade B: Acide 4,4'-[1,16-hexadécanediylbis(carbonylamino)] bis(phénylphosphonique)***

*Point de fusion: 245 °C*

| *Microanalyse élémentaire:* | | | |
|---|---|---|---|
| | *C%* | *H%* | *N%* |
| *calculé* | *57,69* | *7,42* | *4,48* |
| *trouvé* | *57,65* | *7,45* | *4,49* |

***Exemple 34**: Acide 4,4'-[1,8-octanediylbis(carbonylamino)]bis(benzylphosphonique)*

Produits de départ: Chlorure de sébacoyle et diéthylester de l'acide 4-amino benzylphosphonique

***Stade A: Diéthylester de l'acide 4,4'-[1,8-octanediylbis(carbonylamino)] bis(benzylphosphonique)***

*Point de fusion: 178 °C*

| *Microanalyse élémentaire:* | | | |
|---|---|---|---|
| | *C%* | *H%* | *N%* |
| *calculé* | *58,89* | *7,72* | *4,29* |
| *trouvé* | *58,88* | *7,89* | *4,50* |

***Stade B: Acide 4,4'-[1,8-octanediylbis(carbonylamino)]bis(benzylphosphonique)***

*Point de fusion: > 260 °C*

| *Microanalyse élémentaire:* | | | |
|---|---|---|---|
| | *C%* | *H%* | *N%* |
| *calculé* | *53,33* | *6,34* | *5,18* |
| *trouvé* | *53,18* | *6,46* | *4,94* |

***Exemple 35**: Acide 4,4'-[1,18-octadécanediylbis(carbonylamino)]bis(phénylphosphonique)*

Produits de départ: Chlorure de l'acide 1,20-eicosanedioique et diéthylester de l'acide 4-aminophénylphosphonique (préparation A).

***Stade A: Diéthyl ester de l'acide 4,4'[1,18-octadécanediylbis(carbonylamino)] bis(phénylphosphonique)***

| *Microanalyse élémentaire:* | | | |
|---|---|---|---|
| | *C%* | *H%* | *N%* |
| *calculé* | *62,81* | *8,70* | *3,66* |
| *trouvé* | *62,65* | *8,58* | *3,54* |

***Stade B: Acide 4,4'-[1,18-octadécanediylbis(carbonylamino)]bis(phénylphosphonique)***

*Point de fusion: 240 °C*

| *Microanalyse élémentaire:* | | | |
|---|---|---|---|
| | *C%* | *H%* | *N%* |
| *calculé* | *58,89* | *7,72* | *4,29* |
| *trouvé* | *58,78* | *7,70* | *4,10* |

***Exemple 36**: Acide 3,3'-[1,10-décanediylbis(carbonylamino)]bis(phénylphosphonique)*

Produits de départ: Chlorure de l'acide 1,12-dodécanedioique et diéthylester de l'acide 4-aminophénylphosphonique (préparation A).

***Stade A: Diéthylester de l'acide 3,3'-[1,10décanediylbis(carbonylamino)] bis(phénylphosphonique)***

*Point de fusion: 141-145 °C*

| *Microanalyse élémentaire:* | | | |
|---|---|---|---|
| | *C%* | *H%* | *N%* |
| *calculé* | *58,89* | *7,72* | *4,29* |
| *trouvé* | *59,00* | *7,66* | *4,21* |

***Stade B: Acide 3,3'-[1,10-décanediylbis(carbonylamino)]bis(phénylphosphonique)***

*Point de fusion: 195-197 °C*

| *Microanalyse élémentaire:* | | | |
|---|---|---|---|
| | *C%* | *H%* | *N%* |
| *calculé* | *53,33* | *6,34* | *5,18* |
| *trouvé* | *53,02* | *6,18* | *4,99* |

***Exemple 37**: Monoéthyl ester de l'acide 4,4'-[1,8-octanediylbis(carbonylamino)] bis(phénylphosphonique)*

Le composé du stade A de l'exemple 1 (600 mg, 0,96 mmol) est agité dans un mélange de 6 ml de NaOH 1N et 6 ml d'éthanol à 80 °C durant une nuit. La solution réactionnel est ensuite refroidit dans un bain de glace et on acidifie avec de l'HCl 3N. Le précipité blanc est filtré et recristallisé dans l'éthanol.

*Point de fusion: 191 °C*

| *Microanalyse élémentaire:* | | | |
|---|---|---|---|
| | *C%* | *H%* | *N%* |
| *calculé* | *54,93* | *6,74* | *4,93* |
| *trouvé* | *54,59* | *6,62* | *4,70* |

***Exemple 38**: Acide 4,4'-[2,5-divinylènefuranediylbis(carbonylamino)]bis (phénylphosphonique)*

Produits de départ: Chlorure de l'acide furane-2,5-diacrylique (préparation M) et diéthylester de l'acide 4-aminophénylphosphonique (préparation A).

***Stade A: Diéthylester de l'acide 4,4'-[2,5-divinylènefuranediylbis (carbonylamino)]bis(phénylphosphonique)***

*Point de fusion: 195-202 °C*

| *Microanalyse élémentaire:* | | | |
|---|---|---|---|
| | *C%* | *H%* | *N%* |
| *calculé* | *57,14* | *5,75* | *4,44* |
| *trouvé* | *57,88* | *5,83* | *4,13* |

***Stade B: Acide 4,4'-[2,5-divinylènefuranediylbis(carbonylamino)]bis (phénylphosphonique)***

*Point de fusion: > 300 °C*

| *Microanalyse élémentaire:* | | | |
|---|---|---|---|
| | *C%* | *H%* | *N%* |
| *calculé* | *50,98* | *3,89* | *5,40* |
| *trouvé* | *50,67* | *3,91* | *5,12* |

***Exemple 39**: Acide 4,4'-[2,5-divinylènethiophènediylbis(carbonylamino)]bis (phénylphosphonique)*

Produits de départ: Chlorure de l'acide thiophène-2,5-diacrylique (préparation N) et diéthylester de l'acide 4-aminophénylphosphonique (préparation A).

***Stade A: Diéthylester de l'acide 4,4'-[2,5-divinylènethiophènediylbis (carbonylamino)]bis(phénylphosphonique)***

*Point de fusion: 249-255 °C*

| *Microanalyse élémentaire:* | | | | |
|---|---|---|---|---|
| | *C%* | *H%* | *N%* | *S%* |
| *calculé* | *55,72* | *5,61* | *4,33* | *4,96* |
| *trouvé* | *55,43* | *5,84* | *4,46* | *4,77* |

***Stade B: Acide 4,4'-[2,5-divinylènethiophènediylbis(carbonylamino)]bis (phénylphosphonique)***

*Point de fusion: >300 °C*

| *Microanalyse élémentaire:* | | | | |
|---|---|---|---|---|
| | *C%* | *H%* | *N%* | *S%* |
| *calculé* | *49,44* | *3,77* | *5,24* | *6,00* |

(suite)

| Microanalyse élémentaire: | | | | |
|---|---|---|---|---|
| | C% | H% | N% | S% |
| trouvé | 49,19 | 3,75 | 5,18 | 5,75 |

***Exemple 40**: Acide 4,4'-[2,6-divinylènenaphtalènediylbis(carbonylamino)]bis (phénylphosphonique)*

Produits de départ: Chlorure de l'acide naphtalène-2,6-diacrylique (préparation O) et diéthylester de l'acide 4-aminophénylphosphonique (préparation A).

***Stade A: Diéthylester de l'acide 4,4'-[2,6-divinylènenaphtalènediylbis(carbonylamino)]bis(phénylphosphonique)***

*Point de fusion: 286-290 °C*

| Microanalyse élémentaire: | | | |
|---|---|---|---|
| | C% | H% | N% |
| calculé | 62,61 | 5,84 | 4,06 |
| trouvé | 62,79 | 5,76 | 3,87 |

***Stade B: Acide 4,4'-[2,6-divinylènenaphtalènediylbis(carbonylamino)]bis (phénylphosphonique)***

*Point de fusion: >260 °C*

| Microanalyse élémentaire: | | | |
|---|---|---|---|
| | C% | H% | N% |
| calculé | 58,14 | 4,18 | 4,84 |
| trouvé | 58,14 | 4,23 | 5,17 |

***Exemple 41**: Acide 4,4'-[2,5-diéthylènethiophènediylbis(carbonylamino)]bis (phénylphosphonique)*

***Stade A: Diéthylester de l'acide 4,4'-[2,5-diéthylènethiophènediylbis (carbonylamino)]bis(phénylphosphonique)***

Le produit décrit au stade A de l'exemple 39 est hydrogéné comme décrit dans l'exemple 25 au stade A.
*Point de fusion: 206-212 °C*

| Microanalyse élémentaire: | | | | |
|---|---|---|---|---|
| | C% | H% | N% | S% |
| calculé | 55,38 | 6,20 | 4,31 | 4,93 |
| trouvé | 55,24 | 6,23 | 4,49 | 4,85 |

***Stade B: Acide 4,4'-[2,5-(diéthylènethiophènediylbis(carbonylamino)]bis (phénylphosphonique)***

*Point de fusion: 298-303 °C*

| Microanalyse élémentaire: | | | | |
|---|---|---|---|---|
| | C% | H% | N% | S% |
| calculé | 49,07 | 4,49 | 5,20 | 5,95 |
| trouvé | 48,66 | 4,51 | 5,15 | 5,84 |

***Exemple 42*: Acide 4,4'-[1,11-undécanediylbis (carbonylamino)]bis(benzylphosphonique)**

Produits de départ: Chlorure de l'acide 1,13-tridécanedioique et diéthylester de l'acide 4-aminobenzylphosphonique

***Stade A: Diéthylester de l'acide 4,4'-[1,11-undécanediylbis(carbonylamino)]bis (benzylphosphonique)***

*Point de fusion: 151-156 °C*

| Microanalyse élémentaire: | | | |
|---|---|---|---|
| | C% | H% | N% |
| calculé | 60,51 | 8,12 | 4,03 |
| trouvé | 60,01 | 8,05 | 4,02 |

***Stade B: Acide 4,4'-[1,11-undécanediylbis(carbonylamino)]bis(benzylphosphonique)***

*Point de fusion: 230-236 °C*

| Microanalyse élémentaire: | | | |
|---|---|---|---|
| | C% | H% | N% |
| calculé | 55,29 | 6,92 | 4,84 |
| trouvé | 55,67 | 6,92 | 4,81 |

***Exemple 43*: Acide 4,4'-[2,7-divinylènefluorènediylbis(carbonylamino)]bis (phénylphosphonique)**

Produits de départ: Chlorure de l'acide fluorène-2,7-diacrylique (préparation P) et diéthylester de l'acide 4-aminophénylphosphonique (préparation A).

***Stade A: Diéthylester de l'acide 4,4'-[2,7-divinylènefluorènediylbis(carbonylamino)] bis(phénylphosphonique)***

*Point de fusion: 288-293 °C*

| Microanalyse élémentaire: | | | |
|---|---|---|---|
| | C% | H% | N% |
| calculé | 64,28 | 5,81 | 3,84 |
| trouvé | 64,13 | 5,69 | 3,68 |

***Stade B: Acide 4,4'-[2,7-divinylènefluorènediylbis(carbonylamino)]bis (phénylphosphonique)***

*Point de fusion: > 300 °C*

| Microanalyse élémentaire: | | | |
|---|---|---|---|
| | C% | H% | N% |
| calculé | 60,40 | 4,25 | 4,54 |
| trouvé | 60,27 | 4,25 | 4,40 |

***Exemple 44*: Acide 4,4'-[1,4-divinylènenaphtalènediylbis(carbonylamino)]bis (phénylphosphonique)**

Produits de départ : Chlorure de l'acide naphtalène-1,4-diacrylique (préparation Q) et diéthylester de l'acide 4-aminophénylphosphonique (préparation A).

***Stade A: Diéthylester de l'acide 4,4'-[1,4-divinylènenaphtalènediylbis (carbonylamino)]bis(phénylphosphonique)***

*Point de fusion: > 260 °C*

| *Microanalyse élémentaire:* | | | |
|---|---|---|---|
| | *C%* | *H%* | *N%* |
| *calculé* | *62,61* | *5,84* | *4,06* |
| *trouvé* | *62,24* | *5,81* | *4,03* |

***Stade B: Acide 4,4'-[1,4-divinylènenaphtalènediylbis(carbonylamino)] bis(phénylphosphonique)***

*Point de fusion: >300 °C*

| *Microanalyse élémentaire:* | | | |
|---|---|---|---|
| | *C%* | *H%* | *N%* |
| *calculé* | *58,14* | *4,18* | *4,84* |
| *trouvé* | *58,08* | *4,21* | *4,81* |

***Exemple 45: Acide 4,4'-[1,4-trans-divinylènecyclohexanediylbis(carbonylamino)]bis (phénylphosphonique)***

Produits de départ: Chlorure de l'acide cyclohexane-1,4-trans-diacrylique (préparation R) et acide 4-aminophénylphosphonique (préparation A).

***Stade A: Diéthylester de l'acide 4,4'-[1,4-trans-divinylène cyclohexanediylbis (carbonylamino)]bis(phénylphosphonique)***

*Point de fusion: >300 °C*

| *Microanalyse élémentaire:* | | | |
|---|---|---|---|
| | *C%* | *H%* | *N%* |
| *calculé* | *59,44* | *6,86* | *4,33* |
| *trouvé* | *59,31* | *6,85* | *4,34* |

***Stade B: Acide 4,4'-[1,4-trans-divinylène cyclohexanediylbis(carbonylamino)] bis(phénylphosphonique)***

*Point de fusion: > 300 °C*

| *Microanalyse élémentaire:* | | | |
|---|---|---|---|
| | *C%* | *H%* | *N%* |
| *calculé* | *53,94* | *5,28* | *5,24* |
| *trouvé* | *53,61* | *5,31* | *5,07* |

***Exemple 46: Acide 4,4'-[1,9-nonanediylbis(carbonylamino)]bis(styryl phosphonique)***

Produits de départ: Chlorure de l'acide 1,11 undécanedioique et diéthylester de l'acide 4-amino styryle phosphonique (préparation S).

***Stade A: Diéthylester de l'acide 4,4'-[1,9-nonanediylbis(carbonylamino)]bis(styryl phosphonique)***

*Point de fusion: 161-166 °C*

| *Microanalyse élémentaire:* | | | |
|---|---|---|---|
| | *C%* | *H%* | *N%* |
| *calculé* | *60,86* | *7,59* | *4,06* |
| *trouvé* | *60,81* | *7,62* | *4,10* |

***Stade B: Acide 4,4'-[1,9-nonanediylbis(carbonylamino)]bis(styryl phosphonique)***

*Point de fusion: > 300 °C*

| *Microanalyse élémentaire:* | | | |
|---|---|---|---|
| | *C%* | *H%* | *N%* |
| *calculé* | *56,05* | *6,27* | *4,84* |
| *trouvé* | *55,83* | *6,34* | *4,72* |

***Exemple 47: Acide 4,4'-[1,4-di-(1,3-trans trans butadiénylène)benzènediylbis (carbonylamino)]bis(phényl phosphonique)***

Produits de départ: Chlorure de l'acide benzène-1,4-di-(1,3-*trans,trans*-butadiénylène) dicarboxylique (préparation T) et diéthylester de l'acide 4-aminophényl phosphonique (préparation A).

***Stade A: Diéthylester de l'Acide 4,4'-[1,4-di-(1,3-trans trans butadiénylène)benzènediylbis (carbonylamino)]bis (phényl phosphonique)***

*Point de fusion: 278-282 °C*

| *Microanalyse élémentaire:* | | | |
|---|---|---|---|
| | *C%* | *H%* | *N%* |
| *calculé* | *62,42* | *6,11* | *4,04* |
| *trouvé* | *62,29* | *5,96* | *3,97* |

***Stade B: Acide 4,4'-[1,4-di-(1,3-trans trans butadiénylène)benzènediylbis (carbonylamino)]bis(phényl phosphonique)***

*Point* de fusion: *> 300 °C*

| *Microanalyse élémentaire:* | | | |
|---|---|---|---|
| | *C%* | *H%* | *N%* |
| *calculé* | *57,94* | *4,51* | *4,83* |
| *trouvé* | *57,43* | *4,35* | *4,76* |

***Exemple 48: Acide 4,4'-[1,13-tridécanediylbis (carbonylamino)]bis(styryl phosphonique)***

Produits de départ: Chlorure de 1,15 pentadécanedioique et le diéthylester de l'acide 4-amino styryle phosphonique (préparation S).

***Stade A: Diéthylester de l'Acide 4,4'-[1,13-tridécanediylbis (carbonylamino)]bis(styryl phosphonique)***

*Point de fusion: 111-115° C*

| *Microanalyse élémentaire:* | | | |
|---|---|---|---|
| | *C%* | *H%* | *N%* |
| *calculé* | *62,72* | *8,10* | *3,75* |
| *trouvé* | *62,37* | *8,18* | *3,74* |

***Stade B: Acide 4,4'-[1,13-tridécanediylbis (carbonylamino)]bis(styryl phosphonique)***

*Point de fusion: > 300° C*

| *Microanalyse élémentaire:* | | | |
|---|---|---|---|
| | *C%* | *H%* | *N%* |
| *calculé* | *58,67* | *6,99* | *4,41* |
| *trouvé* | *58,61* | *7,06* | *4,30* |

***Exemple 49: Acide 4,4'-[1,9-nonanediylbis (carbonylamino)]bis(phényléthylphosphonique)***

Produits de départ: Chlorure de 1,11 undécanedioique et diéthylester de l'acide [2-(4-amino phényl) éthyl] phosphonique (préparation U)

***Stade A: Diéthylester de l'acide 4,4'-[1,9-nonanediylbis (carbonylamino)] bis(phényléthyl phosphonique)***

*Point de fusion: 93-95° C*

| *Microanalyse élémentaire:* | | | |
|---|---|---|---|
| | *C%* | *H%* | *N%* |
| *calculé* | *60,51* | *8,12* | *4,03* |
| *trouvé* | *60,28* | *8,22* | *4,06* |

***Stade B: Acide 4,4'-[1,9(nonanediylbis (carbonylamino)]bis(phényléthyl phosphonique)***

*Point de fusion: > 300 ° C*

| *Microanalyse élémentaire:* | | | |
|---|---|---|---|
| | *C%* | *H%* | *N%* |
| *calculé* | *55,67* | *6,92* | *4,81* |
| *trouvé* | *55,36* | *6,98* | *4,76* |

***Exemple 50: Acide 4,4'-[9,10-divinylèneanthracènediylbis(carbonylamino)]bis(phényl phosphonique)***

Produits de départ: Chlorure de l'acide anthracène-9,10-diacrylique (préparation V) et diéthylester de l'acide 4-aminophénylphosphonique (préparation A).

***Stade A: Diéthyl ester de l'acide 4,4'-[9,10-divinylèneanthracènediylbis (carbonylamino)]bis(phényl phosphonique)***

*Point de fusion: > 260 ° C*

| *Microanalyse élémentaire:* | | | |
|---|---|---|---|
| | *C%* | *H%* | *N%* |
| *calculé* | *64,86* | *5,71* | *3,78* |

(suite)

| *Microanalyse élémentaire:* | | | |
|---|---|---|---|
| | *C%* | *H%* | *N%* |
| *trouvé* | *64,22* | *5,65* | *3,80* |

***Stade B: Acide 4,4'-[9,10-divinylèneanthracènediylbis(carbonylamino)]bis(phényl phosphonique)***

*Point de fusion: > 260° C*

| *Microanalyse élémentaire:* | | | |
|---|---|---|---|
| | *C%* | *H%* | *N%* |
| *calculé* | *61,15* | *4,17* | *4,46* |
| *trouvé* | *60,88* | *4,39* | *4,49* |

***Exemple 51: Acide 4,4'-[1,4-divinylènebenzènediylbis(carbonylamino)]bis(benzyl phosphonique)***

Produits de départ: Chlorure de l'acide benzène-1,4-diacrylique (préparation G) et diéthylester de l'acide 4-aminobenzylphosphonique.

***Stade A: Diéthyl ester de l'acide 4,4'-[1,4-divinylènebenzènediylbis (carbonylamino)]bis(benzyl phosphonique)***

*Point de fusion: > 260 °C*

| *Microanalyse élémentaire:* | | | |
|---|---|---|---|
| | *C%* | *H%* | *N%* |
| *calculé* | *61,07* | *6,33* | *4,19* |
| *trouvé* | *60,73* | *6,21* | *4,15* |

***Stade B: Acide 4,4'-[1,4-divinylènebenzènediylbis(carbonylamino)]bis(benzyl phosphonique)***

*Point de fusion: > 300° C*

| *Microanalyse élémentaire:* | | | |
|---|---|---|---|
| | *C%* | *H%* | *N%* |
| *calculé* | *56,12* | *4,71* | *5,03* |
| *trouvé* | *55,72* | *4,87* | *4,83* |

***Exemple 52: Acide 2,2'-[1,8-octanediylbis(carbonylamino)]bis(phénylphosphonique)***

Produits de départ: Chlorure de sébacoyle et diéthylester de l'acide 2-aminophényl phosphonique (préparation W).

***Stade A: Diéthyl ester de l'acide 2,2'-[1,8-octanediylbis(carbonylamino)] bis(phénylphosphonique)***

*Point de fusion: 61-64° C*

***Stade B: Acide 2,2'-[1,8-octanediylbis(carbonylamino)]bis(phénylphosphonique)***

*Point de fusion: 102-105 °C*

| *Microanalyse élémentaire:* | | | |
|---|---|---|---|
| | *C%* | *H%* | *N%* |
| *calculé* | *51,57* | *5,90* | *5,47* |
| *trouvé* | *51,86* | *6,12* | *5,48* |

***<u>Exemple 53</u>: Monoéthyl ester de l'acide 4,4'-[1,9-nonanediylbis(carbonylamino)] bis(phényléthylphosphonique)***

*Préparé par hydrolyse partielle du diéthylester correspondant (produit du stade A de l'exemple 51).*

Le produit du stade A de l'exemple 51 (1 g, 1,44 mmol) est agité une nuit à 80°C dans 20 ml d'un mélange éthanol/eau (1/1). Le milieu réactionnel est ensuite acidifié à température ambiante par addition goutte à goutte de HCl 3N. Il se forme un précipité blanc qui est filtré, rinçé avec un peu d'eau, séché sous vide et recristallisé dans l'éthanol.

*Point de fusion: 230 °C*

| *Microanalyse élémentaire:* | | | |
|---|---|---|---|
| | *C%* | *H%* | *N%* |
| *calculé* | *58,30* | *7,57* | *4,39* |
| *trouvé* | *58,37* | *7,73* | *4,49* |

***<u>Exemple 54</u>: Acide 4,4'-[1,8-octanediylbis(carbonylamino)]bis(phényléthylphosphonique***

<u>Produits de départ</u>: Chlorure de sébacoyle et diéthylester de l'acide [2-(4-amino phényl) éthyl] phosphonique (préparation U)

***Stade A: Diéthylester de l'acide 4,4'-[1,8-octanediylbis(carbonylamino)] bis(phényléthylphosphonique)***

*Point de fusion: 110-114 °C*

| *Microanalyse élémentaire:* | | | |
|---|---|---|---|
| | *C%* | *H%* | *N%* |
| *calculé* | *59,99* | *7,99* | *4,12* |
| *trouvé* | *60,12* | *8,19* | *4,10* |

***Stade B: Acide 4,4'-[1,8-octanediylbis(carbonylamino)]bis(phényléthylphosphonique)***

*Point de fusion: >300 °C*

| *Microanalyse élémentaire:* | | | |
|---|---|---|---|
| | *C%* | *H%* | *N%* |
| *calculé* | *54,93* | *6,74* | *4,93* |
| *trouvé* | *54,75* | *6,78* | *4,87* |

***<u>Exemple 55</u>: Acide 4,4'-[1,10-décanediylbis(carbonylamino)]bis(phényléthyl phosphonique)***

<u>Produits de départ:</u> Chlorure de 1,12-dodécanedioique et diéthylester de l'acide [2-(4-amino phényl) éthyl] phosphonique (préparation U)

***Stade A: Diéthylester de l'acide 4,4'-[1,10-décanediylbis(carbonylamino)] bis(phényléthylphosphonique))***

*Point de fusion: 133-138 °C*

| *Microanalyse élémentaire:* | | | |
|---|---|---|---|
| | *C%* | *H%* | *N%* |
| *calculé* | *61,00* | *8,25* | *3,95* |
| *trouvé* | *61,07* | *8,47* | *3,94* |

***Stade B: Acide 4,4'-[1,10-décanediylbis(carbonylamino)]bis(phényléthyl phosphonique))***

*Point de fusion: > 300° C*

| *Microanalyse élémentaire:* | | | |
|---|---|---|---|
| | *C%* | *H%* | *N%* |
| *calculé* | *56,37* | *7,10* | *4,70* |
| *trouvé* | *56,46* | *7,27* | *4,74* |

***Exemple 56** **Acide 4,4'-(1,10-décanediylbisamino)bis(phénylphosphonique), dibromhydrate***

***Stade A: Diéthylester de l'acide 4,4'-(1,10-décanediylbisamino)bis(phénylphosphonique)***

Le produit du stade A de l'exemple 1 (700 mg, 1,12 mmol) est mis en suspension dans 300 ml de THF à 0°C. On additionne goutte à goutte une solution de $BH_3$-THF (1 M dans THF, 15 ml). On laisse revenir à température ambiante et on poursuit l'agitation une nuit. La solution réactionnelle est traitée avec 10 ml de méthanol et est agitée 3 jours à température ambiante avant d'être concentrée. Le résidu est repris dans du méthanol et on évapore à sec. Le produit est purifié par chromatographie sur silice (dichlorométhane/méthanol; 90:10).
*Point de fusion: 158-159° C*

| *Microanalyse élémentaire:* | | | |
|---|---|---|---|
| | *C%* | *H%* | *N%* |
| *calculé* | *60,39* | *8,45* | *4,69* |
| *trouvé* | *60,33* | *8,47* | *4,64* |

***Stade B: Acide 4,4'-(1,10-décanediylbisamino)bis(phénylphosphonique), dibromhydrate***

Le produit du stade A (320 mg, 0,536 mmol) est agité 1 heure à reflux dans l'acétonitrile en présence de 1,70 ml (6,43 mmol) de bromotriméthylsilane. On évapore à sec, reprend dans 150 ml de méthanol et agite une nuit à température ambiante. On évapore à sec. Le résidu est trituré dans l'éther et on filtre le produit attendu.
*Point de fusion: 190-192 ° C*

| *Microanalyse élémentaire:* | | | | |
|---|---|---|---|---|
| | *C%* | *H%* | *N%* | *Br%* |
| *calculé* | *40,89* | *5,61* | *4,33* | *24,73* |
| *trouvé* | *41,23* | *5,68* | *4,50* | *22,90* |

***Exemple 57**: **Acide 4,4'-[1,10-décanediylbis(sulfonylamino)]bis(phénylphosphonique)***

Produit de départ : Chlorure de l'acide 1,10-décanedisulfonique et composé décrit dans la préparation A.

***Stade A: Diéthylester de l'acide 4,4'-[1,10-décanediylbis(sulfonylamino)] bis(phénylphosphonique).***

***Stade B: Acide 4,4'-[1,10-décanediylbis(sulfonylamino)]bis(phénylphosphonique)***

## Etude pharmacologique des composés de l'invention

### Exemple 58: Cytotoxicité des composés de l'invention

Trois lignées cellulaires ont été utilisées :

- 1 leucémie murine, L1210,
- 1 carcinome épidermoide humain, A431,
- 1 culture primaire de cellules endothéliales d'aorte de porc, CEAP.

Les cellules sont cultivées dans du milieu de culture RPMI 1640 complet contenant 10 % de sérum de veau foetal, 2 mM de glutamine, 50 unités/ml de pénicilline, 50 µg/ml de streptomycine et 10 mM d'HEPES (pH = 7,4).
Les cellules sont réparties dans des microplaques et exposées aux composés de l'invention. Les cellules sont ensuite incubées pendant deux jours (L1210), 3 jours (CEAP), et 4 jours (A431). Le nombre de cellules viables est ensuite quantifié par un essai colorimétrique, le Microculture Tetrazolium Assay (Carmichael J., DeGraff W.G., Gazdar A.F., Minna J.D. and Mitchell J.R., Evaluation of a tetrazolium-based semiautomated colorimetric assay : assessment of chemosensitivity testing, *Cancer Res.,* 47, 936-942, (1987)).

Ce test a montré que les composés de l'invention sont démunis de pouvoir cytotoxique sur les trois lignées étudiées. Les IC50 (concentrations en produit testé qui inhibent à 50 % la prolifération des cellules traitées) sont comprises entre 100 et 750 µM.

### Exemple 59 : Inhibition de la néovascularisation de la membrane chorio-allantoïdienne d'embryon de poulet (Test de CAM)

Ce test est réalisé avec des embryons de poulet comme décrit précédemment (Crum R., Szabo S. and Folkman J., *Science,* (1985), 230, 1375-1378). Les oeufs fécondés (J0) sont incubés à 37°C. Une poche d'air est créée en prélevant 1 ml d'albumine (J3), puis une fenêtre est découpée dans la coquille (J4) et la membrane vitelline est enlevée pour dégager la membrane chorio-allantoïdienne (CAM).

Les produits à tester sont solubilisés dans de l'éthanol et déposés sur des disques de méthylcellulose qui sont séchés et déposés sur la MCA au jour 6. Entre 8 et 16 oeufs sont utilisés par groupe. La zone située autour du disque est ensuite examinée 48 heures plus tard. Les oeufs présentant une zone avasculaire supérieure à 4 mm de diamètre sont dénombrés et les résultats sont exprimés en pourcentage d'oeufs présentant une zone avasculaire. Les composés de l'invention ont montré dans ce test un pouvoir inhibiteur de la néovascularisation supérieur à celui de la suramine. Les résultats sont regroupés dans le tableau suivant :

| Exemple | Inhibition (%) de la néovascularisation dose (125 µM) |
|---|---|
| 1 | 64 |
| 5 | 68 |
| 7 | 56 |
| 8 | 90 |
| 11 | 65 |
| 24 | 82 |
| 32 | 80 |
| 39 | 75 |

| Exemple (suite) | Inhibition (%) de la néovascularisation dose (125 µM) |
|---|---|
| 40 | 68 |
| 44 | 80 |
| 45 | 89 |
| 47 | 93 |
| Suramine | 20 |

***Exemple 60** : Inhibition de la croissance tumorale in vivo dans le sarcome M 5076 chez la souris*

Les cellules malignes ($10^6$ / souris) sont inoculées au jour 0 par voie sous-cutanée pour former une nodule tumorale. Les souris sont ensuite distribuées au hasard en groupes traités/témoin (12 souris/groupe). Les composés de l'invention sont administrés par voie intrapéritonéale du jour 1 au jour 12. L'inhibition de la croissance tumorale ($I_{CT}$) est évaluée le 13ème jour selon le calcul suivant :

$$\% I_{CT} = 100 - \left( \frac{\text{Volume tumoral médian des souris traitées}}{\text{Volume tumoral médian des souris témoin}} \times 100 \right)$$

L'ensemble des résultats obtenus pour les composés des exemples 1 et 11 sont regroupés ci-dessous :

| Exemples | Test de CAM | Cytotoxicité | | Sarcome M 5076 % $I_{CT}$ |
|---|---|---|---|---|
| | | CEAP | L 1210 | |
| | | $IC_{50}$ µM | $IC_{50}$ µM | |
| 1 | 64 ± 6 | > 500 | > 500 | 61 (60 mg/kg) |

(suite)

| Exemples | Test de CAM | Cytotoxicité | | Sarcome M 5076 % $I_{CT}$ |
|---|---|---|---|---|
| | | CEAP | L 1210 | |
| | | $IC_{50}$ µM | $IC_{50}$ µM | |
| 11 | 65 ± 8 | 429 | > 500 | 60 (30 mg/kg) |
| Suramine | 20 ± 3 | 202 ± 10 | 131 ± 21 | 67 (100 mg/kg) (j 1,4,7 i.v.) |

Ainsi, contrairement à la suramine qui montre une faible inhibition de la néovascularisation (CAM) et une relative cytotoxicité sur la lignée leucémique L 1210, les composés des exemples 1 et 11 ont montré une très bonne corrélation entre l'activité antitumorale et l'inhibition de l'angiogénèse sans effet cytotoxique.

***Exemple 61* : Composition pharmaceutique**

Formule de préparation pour 1000 comprimés dosés à 10 mg

| | |
|---|---|
| Composé de l'exemple 2 | 10 g |
| Hydroxypropylcellulose | 2 g |
| Amidon de blé | 10 g |
| Lactose | 100 g |
| Stéarate de magnésium | 3 g |
| Talc | 3 g |

**Revendications**

**1.** Composé de formule (I) :

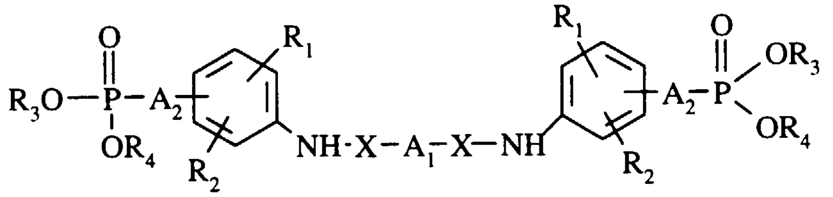

(I)

dans laquelle :

$R_1$,$R_2$ identiques ou différents, représentent un atome d'hydrogène, d'halogène, un groupement alkyle ($C_1$-$C_6$) linéaire ou ramifié, un groupement alkoxy ($C_1$-$C_6$) linéaire ou ramifié, un groupement nitro ou un groupement trihalogénométhyle,

X représente CO, $SO_2$ ou $CH_2$,

$A_1$ représente une chaîne alkylène ($C_1$-$C_{20}$) linéaire ou ramifiée comportant de 0 à 6 doubles liaisons et dans laquelle un ou plusieurs groupements $-CH_2-$ de la chaîne sont éventuellement remplacés par l'un quelconque des groupements suivants :

- phénylène substitué ou non,
- naphtylène substitué ou non,
- anthracénylène substitué ou non,
- cycloalkylène ($C_3$-$C_7$),

- 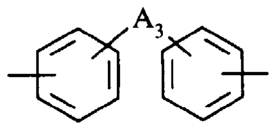

  dans lequel $A_3$ représente $-(CH_2)_m$
  (dans lequel m représente 0, 1 ou 2), -(CH = CH)- ou $SO_2$,

- 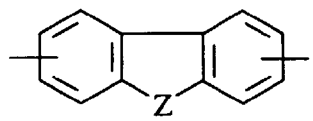

  dans lequel Z représente O, S ou $NR_5$
  (dans lequel $R_5$ représente un atome d'hydrogène ou un groupement alkyle ($C_1$-$C_6$) linéaire ou ramifié),

- 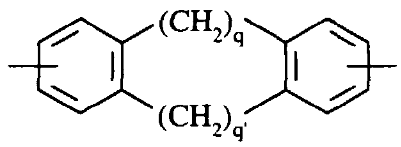

  - dans lequel q, q', identiques ou différents, représentent 0, 1 ou2,

- 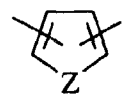

  dans lequel Z est tel que défini précédemment,

$A_2$ représente :

- $-(CH_2)_n-$ dans lequel n est égal à 0, 1, 2 ou 3,
- ou, -CH=CH-,

$R_3$, $R_4$, identiques ou différents, représentent un atome d'hydrogène ou un groupement alkyle ($C_1$-$C_6$) linéaire ou ramifié,

ses isomères ainsi que ses sels d'addition à un acide ou à une base pharmaceutiquement acceptable.

**2.** Composé de formule (I) selon la revendication 1 tel que X représente CO.

**3.** Composé de formule (I) selon la revendication 1 tel que X représente $SO_2$.

**4.** Composé de formule (I) selon la revendication 1 tel que X représente $CH_2$.

**5.** Composé de formule (I) selon la revendication 1 tel que $A_1$ représente un groupement alkylène ($C_1$-$C_{20}$) linéaire ou ramifié.

**6.** Composé de formule (I) selon la revendication 1 dans lequel $A_1$ représente une chaîne alkylène ($C_1$-$C_{20}$) linéaire ou ramifiée comportant 0 à 6 doubles liaisons et dans laquelle un ou plusieurs groupements $-CH_2-$ de la chaîne sont remplacés par un groupement phénylène, naphtylène, cycloalkylène ($C_3$-$C_7$),
ou

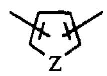

(dans lequel Z est tel que défini dans la revendication 1).

**7.** Composé de formule (I) selon la revendication 1 dans lequel $A_1$ représente une chaîne alkylène ($C_1$-$C_{20}$) dans laquelle un groupement $-CH_2-$ de la chaîne est remplacé par un groupement

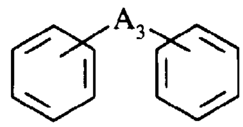

(dans lequel $A_3$ est tel que défini dans la revendication 1).

**8.** Composé de formule (I) selon la revendication 1 dans lequel $A_2$ représente $-(CH_2)_n-$ dans lequel n est égal à 0, 1, 2 ou 3.

**9.** Composé de formule (I) selon la revendication 1 dans lequel $A_2$ représente -CH=CH-.

**10.** Composé de formule (I) selon la revendication 1 qui est l'acide 4,4'-[1,8-octanediylbis(carbonylamino)]bis(phénylphosphonique), ainsi que ses sels d'addition à une base pharmaceutiquement acceptable.

**11.** Composé de formule (I) selon la revendication 1 qui est l'acide 4,4'-[4,4'-diphénylméthanediylbis(carbonylamino)] bis(phénylphosphonique), ainsi que ses sels d'addition à une base pharmaceutiquement acceptable

**12.** Composé de formule (I) selon le revendication 1 qui est l'acide 4,4'-[1,4-divinylènebenzènediylbis(carbonylamino)] bis(phénylphosphonique).

**13.** Procédé de préparation du composé de formule (I) caractérisé en ce que l'on utilise comme produit de départ une amine de formule (II) :

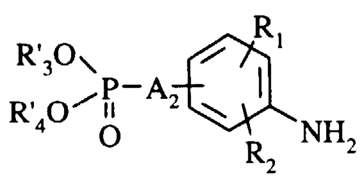

(II)

dans lequel $R_1$, $R_2$ et $A_2$ ont la même signification que dans la formule (I) et $R'_3$, $R'_4$, identiques ou différents, représentent un groupement alkyle ($C_1$-$C_4$) linéaire ou ramifié,
que l'on fait réagir en milieu basique sur 1/2 équivalent d'halogénure de formule (III) :

$$Hal - X - A_1 - X - Hal \qquad (III)$$

dans laquelle Hal représente un atome d'halogène, X et $A_1$ ont la même signification que dans la formule (I), pour conduire au diester de formule (I/a), cas particulier des composés de formule (I):

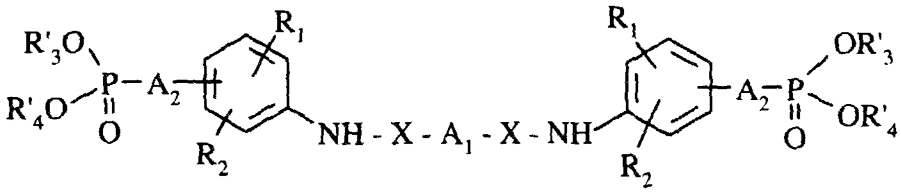

(I/a)

dans laquelle $R_1$, $R_2$, $R'_3$, $R'_4$, X, $A_1$ et $A_2$ sont tels que définis précédemment,
que l'on transforme en monoester correspondant en milieu acide, cas particulier des composés de formule (I),
ou en acide phosphonique correspondant en présence de bromure de triméthylsilyle, cas particulier des composés de formule (I),
diester, monoester ou acide phosphonique,

- qui peut être, le cas échéant, purifié selon une technique classique de purification,
- dont on sépare, le cas échéant, les isomères selon une technique classique de séparation,
- que l'on transforme, si on le souhaite, en ses sels d'addition à un acide ou à une base pharmaceutique acceptable.

**14.** Compositions pharmaceutiques contenant comme principe actif au moins un composé selon l'une quelconque des revendications 1 à 12, seul ou en combinaison avec un ou plusieurs excipients ou véhicules inertes, non toxiques, pharmaceutiquement acceptables.

**15.** Compositions pharmaceutiques selon la revendication 14 contenant au moins un principe actif selon l'une quelconque des revendications 1 à 12 utiles en tant qu'inhibiteurs d'angiogénèse dans la croissance des tumeurs solides, lors du développement des métastases ainsi que dans les rétinopathies diabétiques.